# EUROPEAN PATENT SPECIFICATION

(11) **EP 3 010 528 B1**
(45) Date of publication and mention of the grant of the patent: **11.09.2019**
(21) Application number: 14813855.5
(22) Date of filing: 11.06.2014
(51) Int. Cl.: A61K 38/20, A61K 39/385, A61K 39/39, A61K 39/395, A61K 39/44, A61P 35/00

(54) **IN VIVO TARGETING OF CELLS WITH LIGAND-CONJUGATED PARTICLES**
IN-VIVO-TARGETING VON ZELLEN MIT LIGANDENKONJUGIERTEN TEILCHEN
CIBLAGE IN VIVO DE CELLULES AVEC DES PARTICULES CONJUGUÉES À UN LIGAND

(30) Priority: 19.06.2013 US 201361837137 P
(43) Date of publication of application: 27.04.2016
(60) Divisional application: 19189418.7
(73) Proprietor: Massachusetts Institute of Technology, Cambridge, MA 02139 (US)
(72) Inventor: IRVINE, Darrell, J., Arlington, MA 02476 (US); ZHENG, Yiran, Singapore 649816 (SG); ZHANG, Yuan, Dorchester MA 02125 (US); KWONG, Brandon, Toronto, Ontario M2P 1K5 (CA)
(74) Representative: Inspicos P/S
(86) International application number: PCT/US2014/042004
(87) International publication number: WO 2014/204762

(56) References cited:
- Kwong, Brandon: "Liposome-Anchored Local Delivery of Immunomodulatroy Agents for Tumor Therapy", Massachusetts Institute of Technology , 10 July 2012 (2012-07-10), XP002765894, Retrieved from the Internet: URL:https://dspace.mit.edu/bitstream/handl e/1721.1/76115/820726387-MIT.pdf?sequence= 2 [retrieved on 2017-01-12]
- BRANDON KWONG ET AL: "Induction of potent anti-tumor responses while eliminating systemic side effects via liposome-anchored combinatorial immunotherapy", BIOMATERI, ELSEVIER SCIENCE PUBLISHERS BV., BARKING, GB, vol. 32, no. 22, 28 March 2011 (2011-03-28), pages 5134-5147, XP028214646, ISSN: 0142-9612, DOI: 10.1016/J.BIOMATERIALS.2011.03.067 [retrieved on 2011-04-02]
- ZHENG, Y ET AL.: 'In Vivo Targeting Of Adoptively Transferred T-Cells With Antibody- And Cytokine-Conjugated Liposomes.' J. CONTROL RELEASE. vol. 172, no. 2, 11 June 2013, pages 426 - 435, XP028759063 DOI: 10.1016/J.JCONREL.2013.05.037
- IRVINE, DJ ET AL.: 'Combining Cell Therapy With Nanotechnology For Enhanced Cancer Immunotherapy.' 16TH INTERNATIONAL SYMPOSIUM ON RECENT ADVANCES IN DRUG DELIVERY SYSTEMS. 03 February 2013 - 06 February 2013, SALT LAKE CITY, UT;, pages 9 - 10, XP008182659
- KWONG, B ET AL.: 'Localized Immunotherapy Via Liposome-Anchored Anti-CD137 + IL-2 Prevents Lethal Toxicity And Elicits Local And Systemic Antitumor Immunity.' CANCER RES. vol. 73, no. 5, 01 March 2013, pages 1547 - 1558, XP055304290 DOI: 10.1158/0008-5472.CAN-12-3343

## Description

### BACKGROUND OF INVENTION

Immunotherapy treatments stimulating a patient's own immune system to attack tumors are beginning to show signs of clinical efficacy, demonstrating that the immune system can be harnessed for cancer therapy even in patients with advanced disease [1-3]. Among many immunotherapy strategies in development, adoptive cell therapy (ACT) with autologous tumor-specific T-cells has shown particularly striking results in recent phase I clinical trials [3, 4]. In this approach, autologous T-cells isolated from tumor biopsies or peripheral blood are treated with cytokine/stimulatory cocktails ex vivo to promote expansion of large numbers of tumor-reactive cells that can be re-infused into the patient, following which the transferred cells can home to disseminated tumor sites and destroy metastatic tumors. ACT therapy using completely autologous patient-derived tumor-infiltrating lymphocytes [4, 5] or patient T-cells transduced with genetically engineered T-cell receptors [3, 6] (TCRs, either exogenous TCR chains or chimeric antigen receptors comprised of synthetic antigen-binding Ig domains fused with TCR signaling components) have been demonstrated to elicit objective response rates in up to 70% of patients with advanced metastatic melanoma [4-7] and dramatic cures in chronic lymphoblastic leukemia [3].

Other strategies in cancer immunotherapy involve targeting tumors with antibody- and cytokine-conjugated liposomes [23, 35].

### SUMMARY OF INVENTION

In accordance with the claims, aspects of the invention provide compositions and methods for enhancing endogenous or adoptively-transferred T-cell responses and include repeated (*e.g.,* at least 2 times, at least 3 times, at least 4 times, at least 5 times, or more) *in vivo* delivery (*e.g*., systemic/intravenous delivery) of agents to tumor- or pathogen-reactive T-cells. Methods of the present disclosure, in some embodiments, employ particles to deliver agents to target cells of interest. For example, methods of the present disclosure may be used to target agents to T cells or other leukocytes, including endogenous T cells and adoptively-transferred T cells. In the context of adoptive cell therapy, adoptively-transferred leukocytes (*e.g*., lymphocytes such as T cells) can be repeatedly stimulated with, for example, supporting adjuvants or other agents, thereby providing continuous supporting signals over prolonged durations that might be necessary for elimination of large tumor or pathogen burdens. Such "re-arming" of leukocytes (*e.g*., lymphocytes such as T-cells) with supporting agents can be achieved by repeated administration of targeting particles. In this manner, adoptively-transferred or endogenous leukocytes (*e.g*., lymphocytes such as T-cells) can be re-stimulated multiple times directly *in vivo.* Particles may target, or may be targeted to, particular cell types (*e.g*., T cells) using cell-specific targeting molecules, such as, for example, ligands or receptors such as antibodies or antibody fragments. In some embodiments, further use of internalizing targeting ligands minimizes the likelihood of immune responses against the particle carrier.

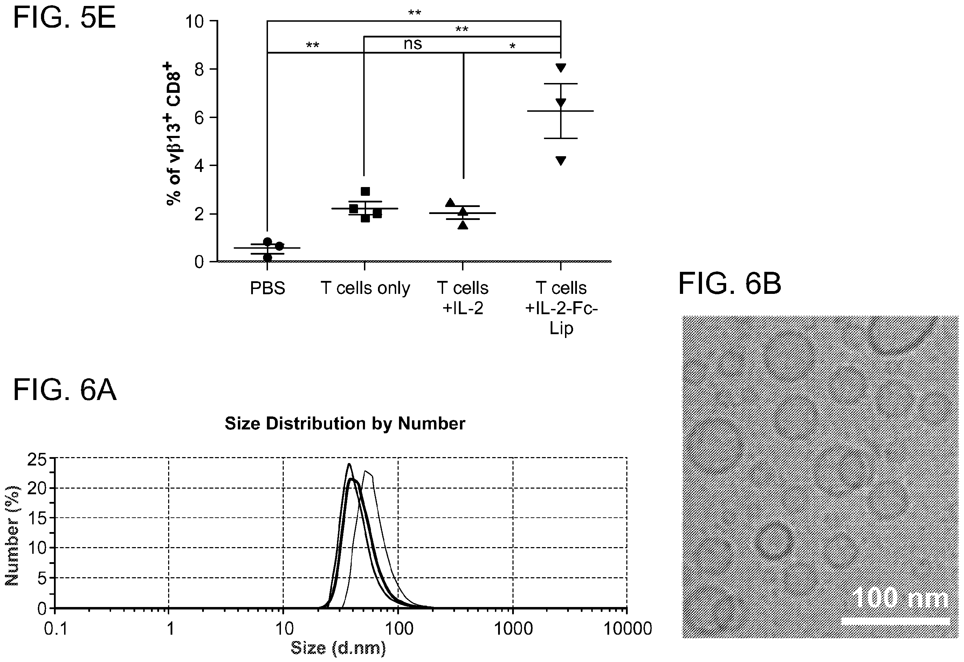

Surprisingly, compositions and methods of the present disclosure permit, in some embodiments, *in vivo* administration of a targeting molecule and/or an agent at a dose higher than otherwise possible if the same targeting molecule and/or an agent were administered in soluble form. For example, as shown in Figures 6A-6C, co-administration of a mixture of soluble forms of anti-CD 137 antibody and IL-2-Fc fusion protein to tumor-bearing subjects resulted in a decrease in tumor volume (FIG. 6A), but the percent survival of the subjects (an indication of toxicity) decreased to about 50% (FIG. 6C). By contrast, co-administration of a mixture of liposomal-conjugated anti-CD137 antibody and liposomal-conjugated IL-2-Fc fusion protein to tumor-bearing subjects, at a dose comparable to the soluble forms (*e.g*., 100 µg anti-CD137, 20 µg IL-2-Fc), resulted in a decrease in tumor volume (FIG. 6A), and a percent survival rate of 100% (FIG. 6C).

Compositions of the present invention are used in therapeutic methods via repeated systemic administration. Accordingly, the present invention relates to lymphocyte-targeting particles (hereinafter also sometimes referred to as particles) that are:
1): a population of lymphocyte-targeting liposomes for use in a therapeutic method in a subject, wherein the population comprises (a) lymphocyte-targeting liposomes comprising a lymphocyte specific ligand, wherein the lymphocyte specific ligand is a cytokine that stimulates lymphocytes; and (b) lymphocyte-targeting liposomes comprising an antibody or antibody fragment that binds to CD137, CTLA-4 or PD-1; and wherein said population of lymphocyte-targeting liposomes is administered to the subject in need thereof by repeated systemic administration.
   or
2): a population of lymphocyte-targeting liposomes for use in a therapeutic method in a subject, wherein the individual lymphocyte-targeting liposomes comprise on their surface (i) a lymphocyte specific ligand, wherein the lymphocyte specific ligand is a cytokine that stimulates lymphocytes; and (ii) an antibody or antibody fragment that binds to CD137, CTLA-4 or PD-1; and wherein said population of lymphocyte-targeting liposomes is administered to the subject in need thereof by repeated systemic administration.

Thus there is employed particles having on their surface targeting molecules (e.g., ligands and/or antibodies or antibody fragments) that are specific for markers on the surface of target cells and, thus, bind to (or are bound by) the target cells. In some embodiments, target cells are leukocytes such as, for example, lymphocytes, including T cells, B cells and NK cells. In some embodiments, target cells are tumor-reactive cells, such as tumor-reactive T cells. In some embodiments, target cells are pathogen-reactive cells.

Targeting molecules (*e.g*., ligands and antibodies, or antibody fragments), in some embodiments, function solely to target the particle to a particular cell. In other embodiments, targeting molecules function solely to stimulate the target cell. In some embodiments, targeting molecules function to target the particle to the cell and to stimulate the cell. An example of such a targeting molecule is the ligand IL-2. Another example of a suitable targeting molecule is an antibody or antibody fragment that binds to CD137. An example of an antibody or antibody fragment that can function to stimulate the target cells is an anti-CTLA4 or an anti-PD-1 antibody or antibody fragment.

Targeting particles (*e.g*., lymphocyte-targeting particles) of the present disclosure may further comprise active agents that act upon the target cells. The nature of the active agent may vary depending on the ultimate outcome that is sought. An example of a class of active agents is inhibitors of immunosuppression. In the context of adoptive cell therapy, such active agents can reduce or eliminate the immunosuppression occurring in or around a tumor, thereby increasing the anti-tumor immune response.

Aspects of the invention provide methods that comprise repeated systemic administration of a population of lymphocyte-targeting particles to a subject, wherein the lymphocyte-targeting particles comprise on their surface at least one lymphocyte-targeting molecule that binds to a lymphocyte cell surface marker. The present disclosure also contemplates, more generally, methods that comprise repeated systemic administration of a population of leukocyte-targeting particles to a subject, wherein the leukocyte-targeting particles comprise on their surface at least one leukocyte-targeting molecule that binds to a leukocyte cell surface marker.

In some embodiments, the at least one lymphocyte-targeting molecule stimulates lymphocytes.

In some embodiments, the at least one lymphocyte-targeting molecule is a lymphocyte-specific ligand that binds to a receptor on the surface of a lymphocyte. In some embodiments, the at least one lymphocyte-targeting molecule is an antibody or an antibody fragment that binds to a cell surface molecule on the surface of a lymphocyte.

In some embodiments, the lymphocyte-targeting particles further comprise an active agent. The active agent may be encapsulated in the lymphocyte-targeting particles, or the active agent may be bound to a surface of the lymphocyte-targeting particles.

In some embodiments, the population comprises (a) lymphocyte-targeting particles comprising a first lymphocyte-targeting molecule and (b) lymphocyte-targeting particles comprising a second lymphocyte-targeting molecule, wherein the second lymphocyte-targeting molecule is different from the first lymphocyte-targeting molecule.

In some embodiments, individual lymphocyte-targeting particles of the population comprise at least two lymphocyte-targeting molecules that are different from each other.

In some embodiments, the lymphocyte-targeting particles target endogenous T cells.

In some embodiments, the lymphocyte-targeting particles comprise an active agent that stimulates activity and/or proliferation of endogenous T cells.

In some embodiments, the lymphocyte-targeting particles target adoptively-transferred T-cells or T cells engineered to express a T cell receptor.

The lymphocyte cell surface marker is CD137, PD-1, or CTLA-4, or or any combination thereof

In some embodiments, the lymphocyte-specific ligand is a cytokine, interleukin, chemokine or growth factor. In some embodiments, the lymphocyte-specific ligand is a cytokine.

In some embodiments, the cytokine is IL-2, IL-7, IL-15, CXCL10, CXCL5, MIP-1a, MIP-1b, or an Fc-fusion protein of any one of the foregoing cytokines.

In some embodiments, the antibody is anti-CD137, anti-CTLA-4, anti-PD-1, or an antibody fragment of any one of the foregoing antibodies.

In some embodiments, the active agent is a chemical entity, a protein, a polypeptide, a peptide, a nucleic acid, a virus-like particle, a steroid, a proteoglycan, a lipid or a carbohydrate.

In some embodiments, the active agent is a therapeutic agent.

In some embodiments, the active agent is an agent that inhibits immunosuppression. For example, the active agent that inhibits immunosuppression may be a Shp1/2 protein tyrosine phosphatase (PTPase) inhibitor.

In some embodiments, the lymphocyte-targeting particles are lymphocyte-targeting liposomes. For example, the lymphocyte-targeting liposomes may be PEGylated lymphocyte-targeting liposomes.

In some embodiments, the lymphocyte-targeting particles are polymer-based lymphocyte-targeting particles.

In some embodiments, repeated administration comprises daily, weekly or biweekly administration.

In some embodiments, the subject has cancer.

In some embodiments, the subject has an infection.

In some embodiments, the lymphocyte-targeting particles are administered parenterally to the subject.

In some embodiments, the subject is undergoing or has undergone adoptive cell therapy.

In some embodiments, the targeting molecule and/or active agent is administered at a dose that is greater than the maximum tolerated dose of a soluble form of the active agent.

Aspects of the invention provide a method comprising repeated administration of the lymphocyte-targeting particles to a subject undergoing adoptive cell therapy, wherein the lymphocyte-targeting particles comprise internally, an active agent.

In another aspect, the invention provides a method comprising repeated administration of particles to a subject undergoing adoptive cell therapy, wherein the particles comprise IL-2 or an IL-2-Fc fusion protein on their surface.

In some embodiments, the administration is not local administration. In some embodiments, the administration is systemic administration.

In some embodiments, the particles internally comprise an active agent. In some embodiments, the active agent is an agent that inhibits immunosuppression. In some embodiments, the agent that inhibits immunosuppression is a Shp1/2 protein tyrosine phosphatase (PTPase) inhibitor.

In some embodiments, the lymphocyte specific ligand is a cytokine. In some embodiments, the lymphocyte specific ligand is IL-2 or an IL-2-Fc fusion protein.

In some embodiments, the lymphocyte cell surface marker is CD137. In some embodiments, the lymphocyte cell surface marker is CTLA-4. In some embodiments, the lymphocyte cell surface marker is PD-1.

In some embodiments, repeated administration comprises daily, weekly, or biweekly administration. In some embodiments, repeated administration comprises administration substantially simultaneously with the administration of tumor-reactive lymphocytes cells, and at least one administration after administration of tumor-reactive lymphocytes. In some embodiments, the tumor-reactive lymphocytes are tumor-reactive T cells. In some embodiments, the tumor-reactive T cells are tumor-reactive CD8+ T cells.

In some embodiments, the adoptive cell therapy comprises administration of tumor-reactive CD8+ T cells.

In another aspect, the invention provides a method comprising repeated administration of the lymphocyte-targeting particles to a subject, wherein the lymphocyte-targeting particles comprise
internally, an active agent.

In another aspect, the invention provides a method comprising repeated administration of particles to a subject, wherein the particles comprise IL-2 or an IL-2-Fc fusion protein on their surface.

In some embodiments, the particles internally comprise an active agent. In some embodiments, the active agent is an agent that inhibits immunosuppression. In some embodiments, the agent that inhibits immunosuppression is a Shp1/2 protein tyrosine phosphatase (PTPase) inhibitor.

In some embodiments, the lymphocyte specific ligand is a cytokine. In some embodiments, the lymphocyte specific ligand is IL-2 or an IL-2-Fc fusion protein.

In some embodiments, the lymphocyte cell surface marker is CD137, CTLA-4 or PD-1.

In some embodiments, the particles are liposomes, including PEGylated liposomes, having on their surface either IL-2 (*e.g*., in the form of an IL-2-Fc fusion), and optionally comprising Shp1/2 PTPase inhibitor.

In some embodiments, repeated administration comprises daily, weekly or biweekly administration.

In some embodiments, the particles target endogenous T cells. In some embodiments, the particles comprise an agent that stimulates activity and/or proliferation of endogenous T cells.

In some embodiments, the subject has an infection. In some embodiments, the subject has a cancer.

In some embodiments, the particles are liposomes. In some embodiments, the liposomes are PEGylated liposomes. In some embodiments, the particles are polymer-based particles.

In some embodiments, the particles are administered parenterally. The particles are typically formulated and thereby administered without cells (*e.g*., such formulations do not contain cells that act as carriers for the particles).

### BRIEF DESCRIPTION OF DRAWINGS

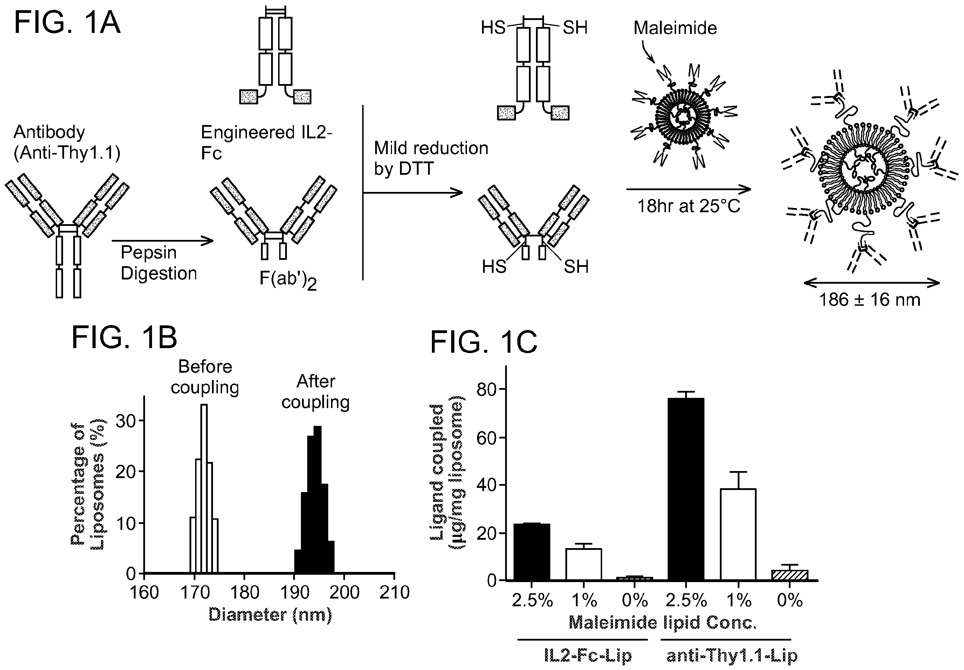

FIGs. 1A-1C show T-cell-targeted liposome synthesis and characterization. (A) Schematic of immunoliposome preparation. (B) Typical particle size distributions for liposomes before antibody conjugation (open bars) and after conjugation (black filled bars) determined by dynamic light scattering. (C) Quantification of ligand (IL-2 cytokine equivalent or anti-Thy1.1) coupled to liposomes incorporating different mole fractions of maleimide-PEG lipid: 2.5% (black filled bar), 1% (open bar) or 0% (striped bar), assessed by IL-2 ELISA and measuring FITC-labeled anti-Thy1.1 incorporation respectively.

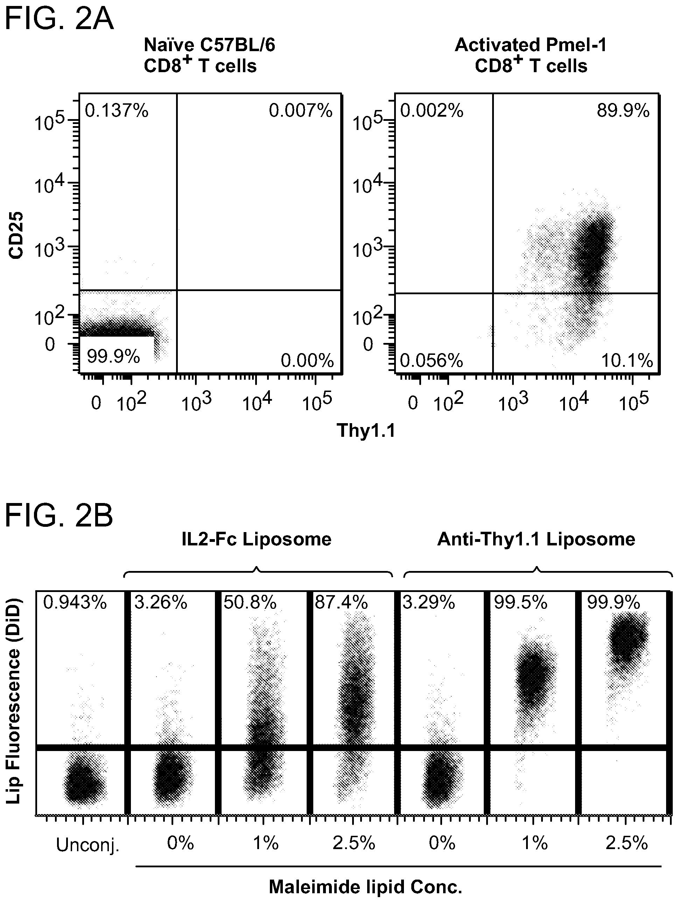

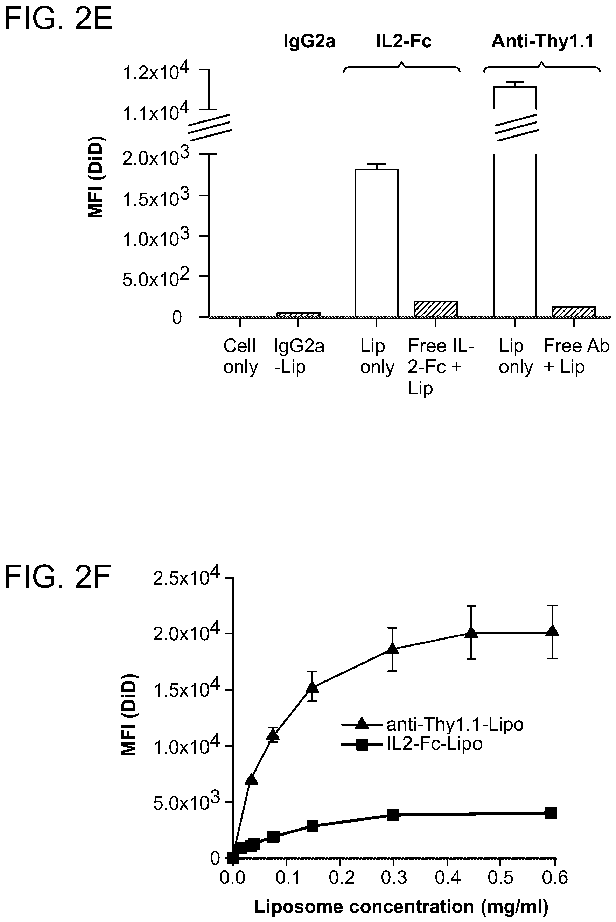

FIGs. 2A-2F show *in vitro* binding of IL-2-Fc-Lip and anti-Thy1.1 F(ab')2 -Lip to primary T-cells. (A) Flow cytometry analysis of cell surface expression of CD25 and Thy1.1 on naive C57BL6/J (Thy1.1-) splenocytes vs. activated pmel-1 Thy1.1+CD8+ T-cells. (B, C) Pmel-1 CD8+ Thy1.1+ T-cells were incubated with 0.7 mg/ml (per 15×10⁶ cells) DiD-labeled liposomes (IL-2-Fc or anti-Thy1.1 F(ab')2 conjugated) for 30 min at 37 °C in complete RPMI, then analyzed by flow cytometry for liposome binding. Shown are representative flow cytometry scatter plots (B) and quantification of Mean Fluorescence Intensity (MFI) of pmel-1 T-cells as a function of mol% of mal-PEG-DSPE included in the vesicles (C). (D, E) Activated pmel-1 CD8+ T-cells were mixed with naive C57BL/6 splenocytes in a 1:1 ratio and incubated with 0.07 mg/ml IL-2-Fc-Lip or 0.15 mg/ml anti-Thy1.1-Lip for 30 min at 37 °C, then analyzed by flow cytometry. (D) Shown are scatter plots representing liposome fluorescence on naive C57BL/6 CD8+ T cells (Thy1.1-) and activated pmel-1 CD8+ T-cells (Thy1.1+) with/without 0.24 mg/ml soluble IL-2-Fc or 1.34 mg/ml anti-Thy1.1 antibody added for 30 min prior to addition of liposomes. Cells incubated with 0.15 mg/ml IgG2a-lipo are also shown. (E) Quantification of the MFI of Pmel-1 CD8+ T cells when bound with respective liposomes or pre-blocked by free Ab/IL-2. (F) Titrated concentrations of fluorescent liposomes were added to 5 ×10⁶ activated pmel-1 T-cells and incubated at 37°C for 30 min, then analyzed by flow cytometry for MFI of T-cell-associated liposomes. *, p<0.05; **, p<0.01; ***, p<0.001.

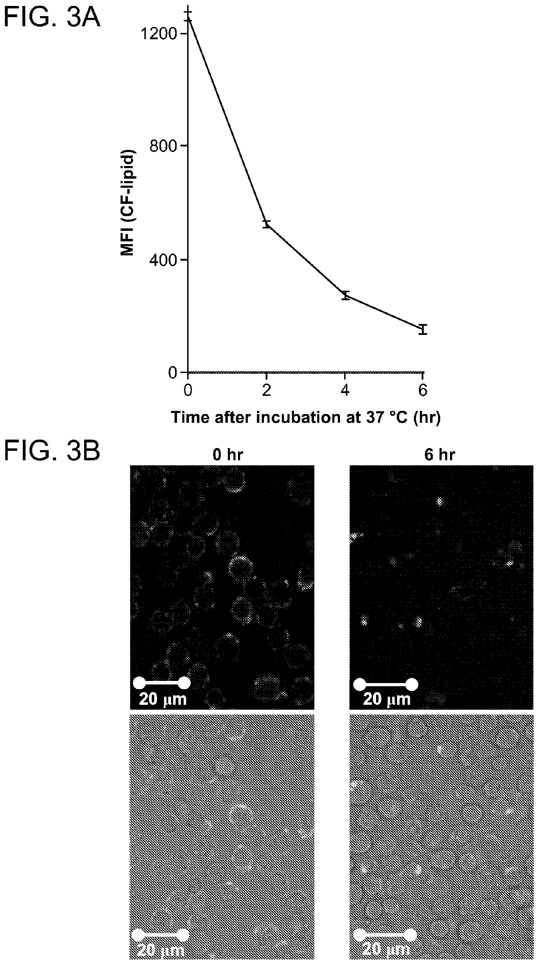

FIG. 3 shows internalization of Thy1.1-targeted liposomes. Carboxy-fluorescein (CF)-labeled anti-Thy1.1-Lip (1.4 mg/ml) were incubated with 12x10⁶ activated pmel-1 CD8+ T-cells in 500 µl RPMI containing 10% FCS for 1 hr at 4°C, washed, then incubated in RPMI at 37°C until analysis by flow cytometry 2 hr, 4 hr or 6 hrs later. (A) MFI of T-cell-associated CF fluorescence. (B) Confocal images of cells at time zero or after 6 hr at 37°C. Scale bar= 20 µm.

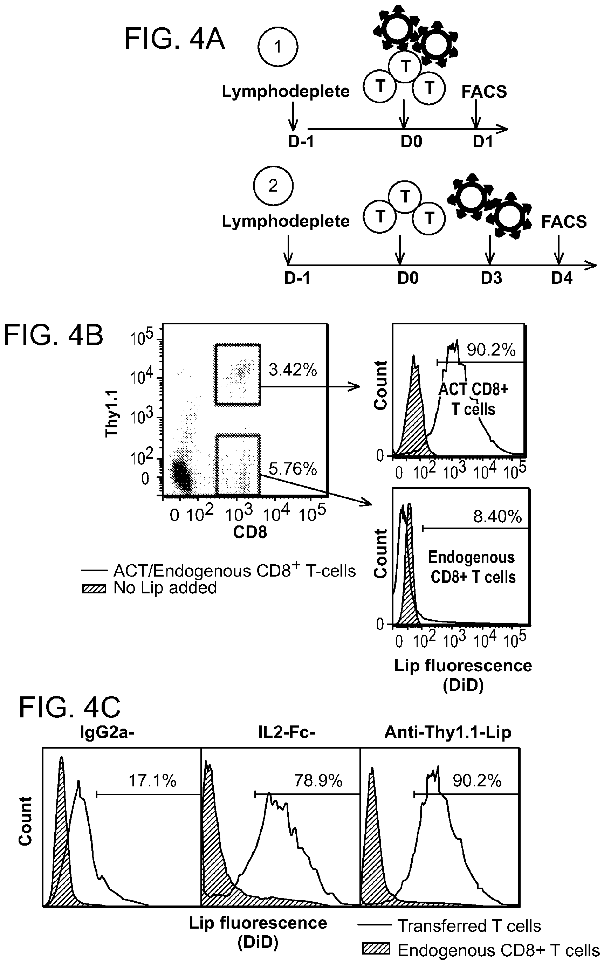

FIGs. 4A-4F show that IL-2-Fc- and anti-Thy1.1-Liposomes target transferred T-cells *in vivo.* C57B1/6 mice received i.v. adoptive transfer of 15x10⁶ pmel-1 CD8+Thy1.1+ T-cells, followed by i.v. injection of 1.4 mg IL-2-Fc-Lip, anti-Thy1.1-Lip, or isotype control IgG2a-Lip either immediately after the T-cells or 3 days after the T-cells. Liposome binding to cells recovered from lymphoid organs and blood was analyzed 24 hr after liposome injections by flow cytometry. (A) Timeline of injections and analysis. (B) Representative flow cytometry plots illustrating gating strategy for analysis of liposome binding to transferred pmel-1 T-cells or endogenous CD8+ T-cells. (C) Representative histograms of pmel-1 T-cell or endogenous CD8+ T-cell labeling following day 0 liposome injections. (D-F) Quantification of percentages of endogenous or transferred T-cells labeled by day 0 or day 3 liposome injections in the blood (D), lymph nodes (E), and spleen (F). n=5 animals/group for IgG2a-Lip and anti-Thy1.1-Lip and n=3 for IL-2-Fc-Lip. *, p<0.05; **, p<0.01; ***, p<0.001.

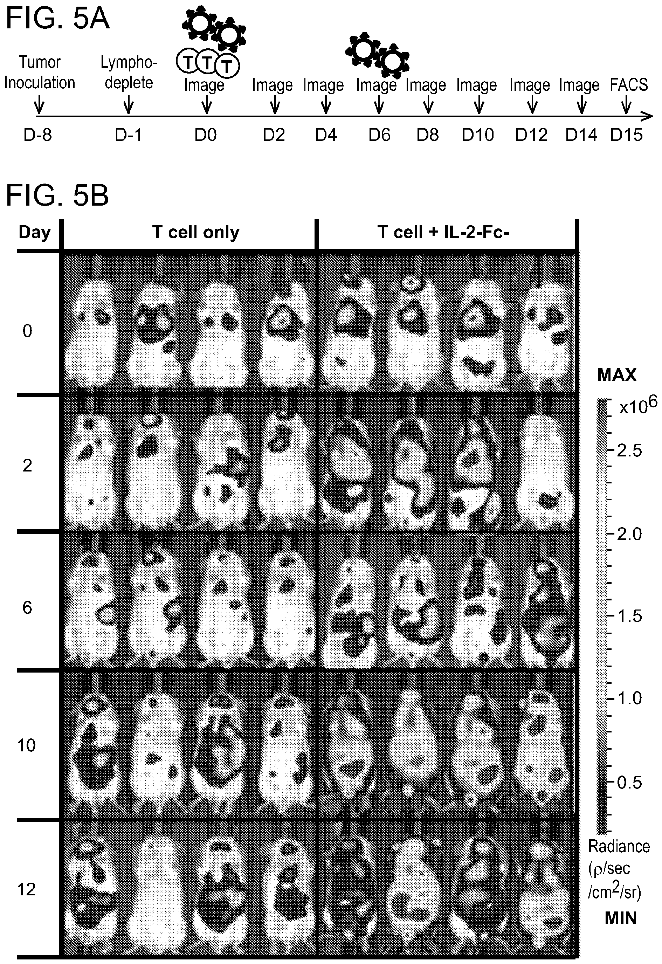

FIGs. 5A-5E show that IL-2-Fc-liposomes allow repeated expansion of target ACT T-cells *in vivo* in tumor-bearing animals. (A-C) B16F10 tumor cells (1x10⁶) were injected i.v. into albino C57B1/6 mice and allowed to establish lung metastases for 7 days. Animals were then sublethally lymphodepleted by irradiation and received i.v. adoptive transfer of 12x10⁶ luciferase-expressing pmel-1 CD8+ T-cells the next day. One group of mice additionally received injections of IL-2-Fc-Lip (1 mg, carrying 60 µg IL-2-Fc or 20 µg IL-2 cytokine equivalent) i.v. immediately after T-cell transfer and again on day 6. (A) Timelines of cell/liposome injections and bioluminescence imaging of T-cells. (B) Representative bioluminescent images of ACT T-cells over time. (C) Quantification of average whole-body T-cell bioluminescence over time. (D-E) Groups of C57B/6 mice with established lung metastases were left untreated or were treated with T-cells as in A, then received either IL-2-Fc-Lip or equivalent total doses of systemic free IL-2 (10 µg day 0,20 µg day 6) injected i.v. on day 0 and day 6. (D) Sample flow cytometry analyses showing percentages of tumor-specific (vβ13 TCR+) CD8+ T-cells among T-cells in inguinal lymph nodes on day 12 after adoptive transfer. (E) Quantification of average frequency of tumor-specific (vβ13 TCR+) CD8+ T-cells in inguinal lymph nodes 12 days after adoptive transfer. n=3-4 animals/group. *, p<0.05; **, p<0.01.
FIG. 6A shows a graph representative of particle size of liposomes measured by dynamic light scattering. Medium gray: liposome-Maleimide; Dark gray: liposome-CD137; Light gray: liposome-IL-2-Fc. FIG. 6B shows a cryo-transmission electron microscopy (TEM) image of antibody-conjugated liposomes.

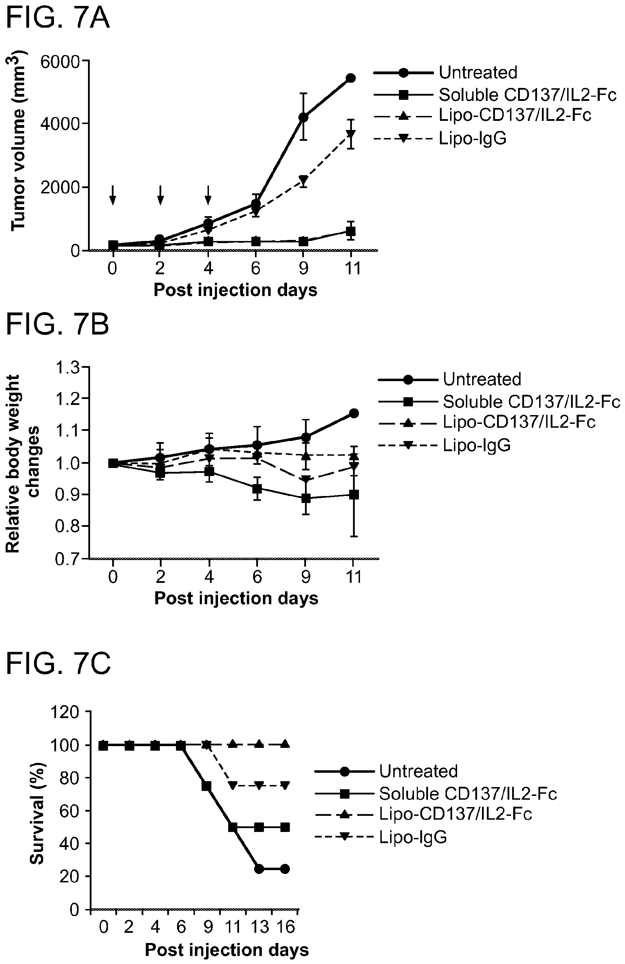

FIGs. 7A-7C show graphs representative of tumor growth inhibition (FIG. 7A), relative body weight changes (normalized to day 0) (FIG. 7B) and a survival curve of the treatment (FIG. 7C) from B16-OVA tumor bearing mice that were given intravenous injections on day 0, 2 and 4 with a 100 µg/dose of CD137 and a 20 µg/dose of IL-2-Fc (untreated, soluble CD137/IL-2-Fc, liposome-conjugated CD137 and liposome-conjugated IL-2-Fc, or liposome-conjugated IgG).

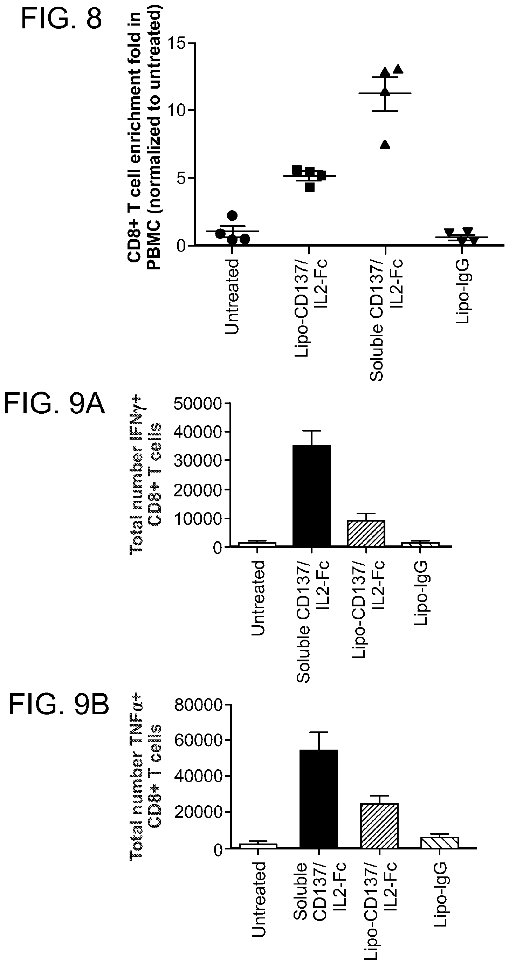

FIG. 8 shows a graph representative of CD8⁺ T cell enrichment in peripheral blood mononucleated cells (PBMCs) on day 6 post injection. CD8+ T cell numbers were analyzed by flow cytometry.
FIGs. 9A-9B show graphs representative of intracellular cytokine staining of IPNγ (FIG. 9A) and TNFα (FIG. 9B) in CD8+ T cells from PBMC. Lymphocytes from PBMC (day 6 post injection) were pulsed with 10 µm OVA protein before analyzed by flow cytometry.

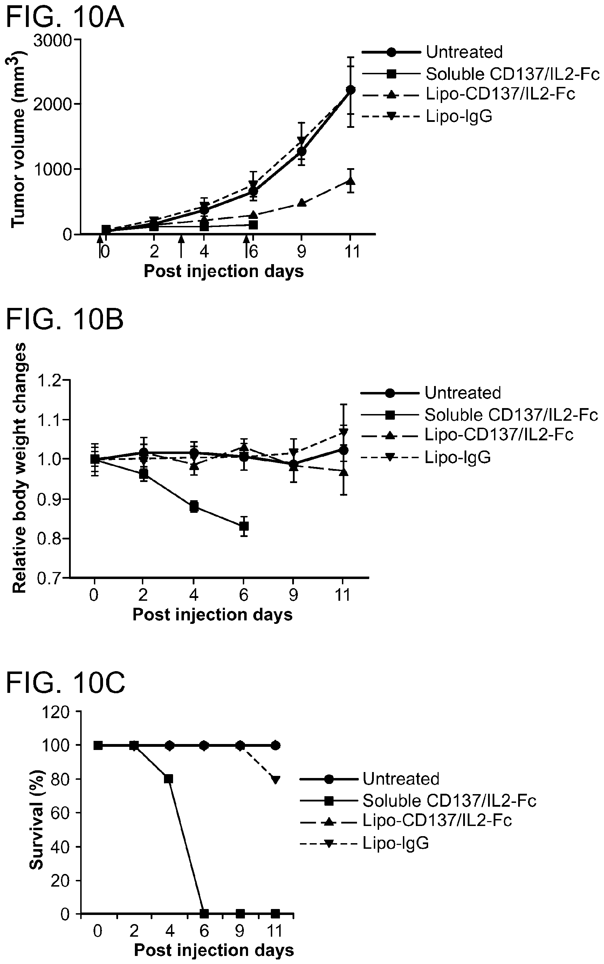

FIGs. 10A-10C show graphs representative of tumor growth inhibition (FIG. 10A), relative body weight changes (normalized to day 0) (FIG. 10B) and survival curve of the treatment (FIG. 10C) obtained from B16F10 tumor bearing mice that were given intravenous injections on day 0, 3 and 6 with a 100 µg/dose of CD137 and a 60 µg/dose of IL-2-Fc.

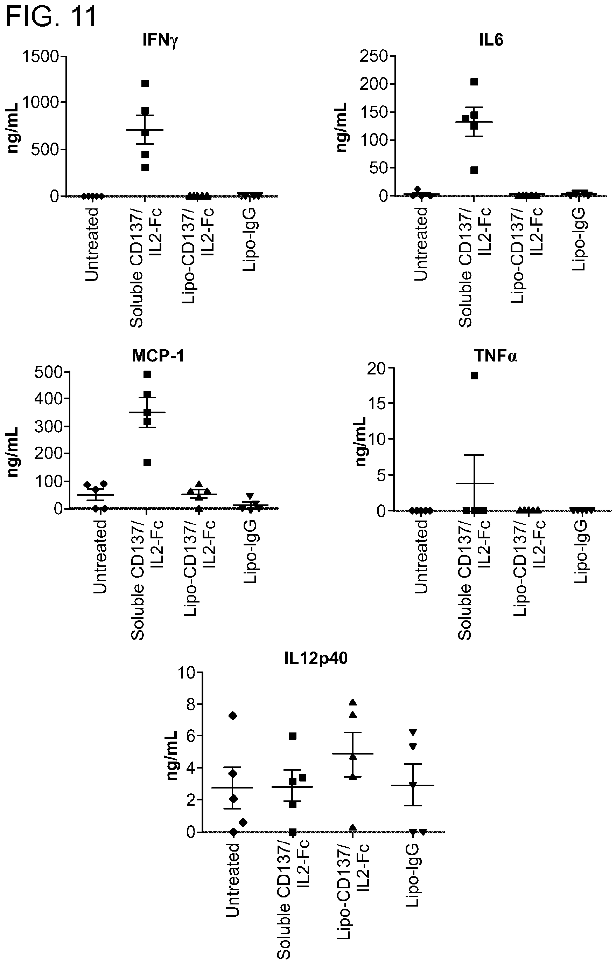

FIG. 11 shows graphs representative of serum cytokine levels obtained from mice after systemic delivery of lipo-CD137/IL-2-Fc, showing prevention of lethal systemic inflammatory toxicity. Two days after single intravenous injection on B16F10 tumor bearing mice, blood serums were collected and serum cytokine levels were measured by LUMINEX® cytokine bead assay.

### DETAILED DESCRIPTION OF INVENTION

Aspects of the invention provide methods for augmenting lymphocyte function *in vivo* by repeated stimulation of lymphocytes *in vivo* using particles as described above that comprise stimulatory agents and/or inhibitors of immunosuppression. In some embodiments, the lymphocytes are adoptively-transferred lymphocytes, such as those used in adoptive cell therapy, which has been used in the treatment of cancer. In some embodiments, the lymphocytes may be endogenous lymphocytes. Aspects of the invention are premised, in part, on the unexpected and, thus, surprising finding that repeated administration of particles according to the invention comprising stimulatory agents and/or inhibitors of immunosuppression augments the activity of target cells *in vivo* more efficiently than systemic administration of stimulatory agents (see for example FIG. 5E). It is therefore contemplated by the invention that the beneficial effects of adoptive cell therapy may be extended in time and augmented in efficacy by boosting the activity and/or proliferation of transferred cells at various times post-transfer. Each administration of particles of the invention to a subject may be regarded as a "boost" since it will result in proliferation of the target cells of interest (and thus expansion of such cell populations), increased longevity of the target cells of interest, and/or increased activity of the target cells of interest.

Provided herein are experimental results evidencing specific targeting of adoptive cell therapy (ACT) T-cells (also referred to herein as adoptively-transferred T cells) *in vivo* using particles in the form of liposomes. Surprisingly, repeated systemic administration to tumor-bearing subjects did not lead to a toxic proinflammatory response, and subjects survived and cleared tumors, indicating that the easier route of administration (systemic, instead of intratumoral) is effective. In these illustrative examples, PEGylated liposomes were conjugated with two types of targeting molecules. The first type of targeting molecule is an antibody against a cell surface antigen expressed by the ACT T-cells. The cell surface antigen may be one that the target cell normally expresses or it may one that the target cell is made to express, for example, through genetic engineering strategies. An example of a cell surface antigen is Thy 1.1. The second type of targeting molecule is a ligand, the receptor for which is found on ACT T cells. One such ligand is interleukin-2 (IL-2). IL-2 binds the trimeric IL-2 receptor (IL-2R) expressed by activated T cells. These targeting molecules provide contrasting targeting strategies: anti-Thy1.1 provides highly specific targeting without overt stimulation of target cells, while IL-2 provides potentially less specific targeting (since IL-2R can be expressed by some endogenous T-cells) but also delivers a direct stimulatory signal to T cells.

Targeting liposomes were shown to label T cells in multiple systemic compartments *in vivo,* with anti-Thy1.1 liposomes binding to >90% of transferred cells following a single systemic injection. Additionally, multiple periodic administrations of targeted stimulatory IL-2-conjugated liposomes resulted in repeated expansion of ACT T-cells *in vivo.*

Accordingly, the aspects of the invention contemplate that these targeted particle strategies can be used to safely amplify the efficacy of ACT while avoiding systemic toxicity associated with many adjuvant drug treatments. Aspects of the invention further contemplate that the strategies are amenable and translatable to other immunotherapy settings, such as enhancement of cancer vaccines and therapeutic interventions in infectious diseases such as human immunodeficiency virus (HIV), which may rely on transferred cells and/or on endogenous cells.

It was also found, surprisingly, that administration of IL-2 conjugated to particles of the invention, and lacking another active agent, resulted in greater expansion of tumor-reactive CD8+ T cells as compared to the same dose of soluble IL-2. As described in greater detail in the Examples, administration of soluble IL-2 at the same dose provided no enhancement in T cell expansion. Accordingly, aspects of the invention also contemplate the use of particles having surface-conjugated IL-2 in expanding T cell populations *in vivo,* including but not limited to tumor-reactive T cells used in adoptive cell therapy (referred to as adoptively-transferred T cells).

### Applications

Methods of the present disclosure embrace the unexpected findings that repeat systemic administration of agents, including targeting molecules, is therapeutically effective when the agents are delivered conjugated to a particle (*e.g*., liposome) and that in so doing, it is possible to administer the agents in a dose that would otherwise be toxic if administered in soluble form. It is to be understood that methods of the invention may be used in a variety of applications in which it is desirable to deliver agents specifically to a target cell population (*e.g*., endogenous T cells and/or adoptively-transferred T cells), and where it is desirable to continually and repeatedly boost an immune response (*e.g*., multiple boosts over the course of several days or weeks). One advantage of the methods of the invention is the ability to deliver active agents to particular cells of interest (*e.g*., lymphocytes), potentially at particular regions of the interest in the body, thereby avoiding the adverse effects associated with simple systemic administration of a soluble active agent.

Methods of the present disclosure may, therefore, be used in subjects undergoing or who have undergone adoptive cell therapy. Typically, such subjects have cancer or are at risk of developing cancer (*e.g.,* they may be in remission or may be genetically or environmentally predisposed to developing cancer).

Methods of the present disclosure, however, may also be used in other applications that require enhanced immune responses, including prolonged enhanced immune responses over a period of time. Non-limiting examples include vaccine-based methods and cell-based methods.

### Target cells

Cells that may be targeted using particles of the invention may be those occurring endogenously in a subject, or those that are transferred (*e.g*., administered) to a subject, for example, for therapeutic or prophylactic benefit. A cell is considered "endogenous" in a subject if it originates from within the subject and has never been removed from the subject. A cell is considered an "adoptively-transferred cell" if it is obtained from a subject and then transferred back into the same subject or if it is obtained from a subject and transferred into a new subject. Adoptively-transferred cells include, for example, autologous subject-derived (*e.g*., human patient-derived) tumor-infiltrating lymphocytes as well as subject T cells transduced with engineered (*e.g*., genetically engineered) T cell receptors (TCRs). T cell receptors may be, for example, exogenous T cell chains or chimeric antigen receptors composed of synthetic antigen-binding Ig domains fused with TCR signaling components. A cell is considered to be "subject-derived" if it is obtained from (*e.g*., isolated from) the subject.

In the context of adoptive cell therapy, the target cells and the transferred cells are typically one and the same in the context of the invention. For example, tumor-reactive CD8⁺ T cells may be transferred to a subject in need of such therapy, and may also be targeted by particles of the invention. The target cells are typically immune cells such as, but not limited, to lymphocytes. Lymphocytes of the present disclosure may be T cells, such as CD8⁺ T cells, B cells or natural killer (NK) cells. In the context of adoptive cell therapy in subjects having cancer, the target cells are tumor-reactive (or tumor-specific) T cells. Transferred cells may be autologous to the subject being treated, or they may be allogeneic.

It is to be understood that any immune cell-based therapy may benefit from methods of the invention, including therapies that involve transfer of dendritic cells, cell-based vaccines, and the like and therapies that involve stimulation of endogenous lymphocytes.

Target cells may be tumor-reactive cells. This means that they recognize and/or bind to tumor cells and/or are involved in an immune response directed against the tumor.

Target cells may be pathogen-reactive cells. This means that they recognize and/or bind to pathogens or pathogen-infected cells and/or are involved in an immune response directed against the pathogen or pathogen-infected cells.

Target cells (*e.g*., lymphocytes) of the present disclosure have cell surface markers that bind to (or are bound by) cognate recognition molecules (*e.g*., lymphocyte-targeting molecules) present on the surface of targeting particles (*e.g*., lymphocyte-targeting particles). A "cell surface marker" refers to a moiety present on the surface of cells that serves as a marker of specific cell types. Cell surface moieties include, without limitation, those used for immunophenotyping cells, such as CD (Classification Determinant) proteins. Other cell surface moieties are contemplated herein. It should be understood that cell-specific targeting molecules present on particles of the present disclosure typically confer cell-specific targeting of the particles. Thus, for example, a liposome conjugated to an anti-CD137 antibody is considered a lymphocyte-targeting particle (and more specifically, a T cell-targeting particle) because anti-CD137 antibody is a lymphocyte-targeting molecule that specifically recognizes and binds to CD137, which is expressed on T cells, thereby targeting the particle to the T cells.

The lymphocyte cell surface markers include, CD137, CTLA-4 and PD-1.

### Targeting Molecules

"Targeting molecules" refers to molecules (*e.g*., ligands, receptors and/or antibodies/antibody fragments) that bind to (*e.g*., bind specifically to) target cells of interest (*e.g*., lymphocytes). A targeting molecule is considered to bind to a target cell if it binds to a cell surface marker (*e.g*., antigen, ligand, receptor) of the target cell. In some embodiments, targeting molecules bind specifically to particular target cells - that is, they bind to cell surface markers that are present only on the particular target cells. Thus, a targeting molecule is considered to bind specifically to a T cell if it binds a cell surface marker that is expressed only on T cells.

In the context of adoptive cell therapy, for example, adoptively-transferred T cells in a subject may uniquely express a cell surface marker (*e.g*., Thy1.1), which itself may be considered, for example, a ligand or a receptor. A cell surface marker that is "uniquely expressed" by a particular cell type is expressed by no other cell types. Thus, "specific binding" occurs, for example, when an anti-Thy1.1 antibody that is conjugated to a T cell-targeting particle binds to Thy1.1 on the surface of T cells. Adoptively-transferred cells may naturally express a unique marker or they may be modified to express a unique marker. Such modification may include, without limitation, genetic engineering of the adoptively-transferred cells.

Targeting molecules (*e.g*., ligands or antibodies) that are bound by (or bind to) lymphocytes are referred to herein as "lymphocyte-targeting molecules." Lymphocyte-targeting molecules include lymphocyte-targeting ligands and lymphocyte-targeting antibodies and antibody fragments such as a Fab fragment.

Ligands that are bound by (or that bind to) lymphocytes may be referred to herein as "lymphocyte-targeting ligands" or "lymphocyte-specific ligands." Examples of lymphocyte-targeting ligands include, without limitation, cytokines, which as used generally herein encompass cytokines, interleukins, chemokines and growth factors. Non-limiting examples of cytokines include IL-2, IL-7, IL-15, CXCL10, CXCL5, MIP-1a and MIP-1b. In some embodiments, the cytokine is IL-2. In some embodiments, a ligand may be in the form of an Fc fusion protein. For example, an IL-2 ligand may be an IL-2-Fc fusion protein. Other non-limiting examples of Fc fusion proteins include IL-7, IL-15, CXCL10, CXCL5, MIP-1a and MIP-1b Fc fusion proteins. Other ligands and Fc fusion proteins are contemplated herein.

Antibodies that are bound by (or that bind to) lymphocytes may be referred to herein as "lymphocyte-targeting antibodies" or "lymphocyte-specific antibodies." Antibody fragments that are bound by (or that bind to) lymphocytes may be referred to herein as "lymphocyte-targeting antibody fragments" or "lymphocyte-specific antibody fragments." The lymphocyte-targeting antibodies include, CD137, CTLA-4 and PD-1. Also contemplated herein are immunostimulatory antibodies including, without limitation, anti-PD-1, anti-CTLA4, anti-PDLl and anti-LaG3 antibodies. Antibody fragments of any of the foregoing antibodies are also contemplated herein. Other antibodies and antibody fragments are contemplated herein. In some embodiments, antibodies used in accordance with the present disclosure are monoclonal antibodies. In some embodiments, antibodies used in accordance with the present disclosure are chimeric antibodies.

It should be understood that a targeting particle of the present invention may comprise at least one (*e.g*., two or more) targeting molecules that are the same as each other (*e.g*., targeting ligands) or different from each other (*e.g*., targeting ligands and targeting antibodies). For example, a lymphocyte-targeting particle may comprise an anti-CD137 antibody and IL-2. Alternatively, a population of lymphocyte-targeting particles may comprise a portion of lymphocyte-targeting particles (*e.g*., half) that comprise one type of lymphocyte-targeting molecule (*e.g*., anti-CD137 antibody), and another portion of lymphocyte-targeting particles that comprise another, different, type of lymphocyte-targeting molecule (*e.g*., IL-2). Thus, mixtures of different targeting particles are contemplated herein. In some embodiments, 10%, 20%, 30%, 40%, 50%, 60%, 70%, 80% or 90% of a mixture comprises one type of lymphocyte-targeting molecule, while the remaining portion or portions of the mixture comprise(s) another type(s) of lymphocyte-targeting molecule(s).

### Targeting Particles

Compositions and methods of the invention involve targeting particles, as defined above. (also referred to as targeted particles). "Targeting particles" refers to particles that comprise on their surface targeting molecules (*e.g*., ligands, receptors and/or antibodies/antibody fragments) that bind to (or are bound by) cell surface markers on target cells of interest, such as lymphocytes (*e.g*., T cells). A targeting particle is considered to comprise a targeting molecule on its surface if the targeting molecule is associated with or interacts with (*e.g*., is covalently or non-covalently conjugated to/bound to) the surface of the targeting particle.

"Particles," as used herein, refer to particulate carriers (*e.g*., are capable of transporting molecules), optionally with active agent encapsulated in or bound to (*e.g*., covalently or non-covalently conjugated to) the particle surface. Particles of the present disclosure include, liposomes.

Targeting particles that are bound by (or bind to) targeting molecules that bind to (*e.g*., bind specifically to) lymphocytes (*e.g*., bind to lymphocyte cell surface markers) are referred to as "lymphocyte-targeting particles."

Particles of the present disclosure may be of any suitable size. As used herein, nanoparticles are particles of approximate nanometer dimensions. As used herein, microparticles are particles of approximate micrometer dimensions. The invention contemplates the use of nanoparticles and/or microparticles.

The diameter of a particle may range from 1-1000 nanometers (nm). In some embodiments, the diameter ranges in size from 20 to 750 nm, from 20 to 500 nm, or from 20 to 250 nm. In some embodiments, the diameter ranges in size from 50 to 750 nm, from 50 to 500 nm, from 50 to 250 nm, or from 100-300 nm. In some embodiments, the diameter is 100, 150, 200 nm, 250 nm or 300 nm. In some embodiments, the diameter ranges in size from about 20 to 750 nm, from about 20 to 500 nm, or from about 20 to 250 nm. In some embodiments, the diameter ranges in size from about 50 to 750 nm, from about 50 to 500 nm, from about 50 to 250 nm, or from about 100-300 nm. In some embodiments, the diameter is about 100, about 150, about 200 nm, about 250 nm or about 300 nm.

In some embodiments, the diameter of a microparticle may range from 0.1 µm to 100 µm (or about 0.1 µm to about 100 µm), 0.1 µm to 90 µm, 0.1 µm to 80 µm, 0.1 µm to 70 µm, 0.1 µm to 60 µm, 0.1 µm to 50 µm, 0.1 µm to 40 µm, 0.1 µm to 30 µm, 0.1 µm to 20 µm, 0.1 µm to 10 µm, 0.1 µm to 5 µm, 0.1 µm to 4 µm, 0.1 µm to 3 µm, 0.1 µm to 2 µm or 0.1 µm to 1 µm.

As used in the context of particle sizes and diameters, the term "about" means +/- 5% of the absolute value stated.

In some embodiments, particles of the present disclosure comprise an active agent and release the active agent over a period of time, ranging from hours to days. The particles may gradually degrade in an aqueous environment, such as occurs *in vivo.* If active agents are dispersed throughout the particles, then release of the active agents will occur as the outermost layers of the particle degrade or as pores within the particle enlarge.

In some embodiments, particles of the present disclosure comprise an active agent and release the active agent all at once as the particle "bursts."

Particles of the present disclosure are administered in a cell-free formulation. This means that they are not bound to cells and are not formulated with cells prior to administration. As described above, particles of the present disclosure may be referred to herein as "lymphocyte-targeting particle." Lymphocyte-targeting particles are able to target lymphocytes *in vivo* without the assistance of carrier cells or other carrier vehicles.

Particles of the present disclosure may be endocytosed when used *in vivo,* although methods of the invention are not dependent upon endocytosis of the particles.

In some embodiments, particles are porous particles. In some embodiments, particles are hollow core particles. Particles of the present disclosure are not viruses or particles thereof (*e.g*., virus-like particles (VLPs)). Particles of the present disclosure, in some embodiments, are biodegradable and, thus, typically are not magnetic. Biodegradable particles may be synthesized using methods known in the art including, without limitation, solvent evaporation, hot melt microencapsulation, solvent removal and spray drying. Exemplary methods for synthesizing particles are described in Bershteyn et al., Soft Matter 4:1787-1787, 2008 and in US 2008/0014144 A1, the specific teachings of which relating to particle synthesis are incorporated herein by reference.

Particles of the present disclosure are lipid-based particles, such as liposomes. They may be natural or synthetic polymer-based particles having a lipid coating. In some embodiments, particles of the invention are multilamellar lipid vesicles (*e.g*., interbilayer-crosslinked multilameller lipid vesicles) (*e.g.,* Moon et al., Nature Materials 10, 243-251 (2011)).

### Lipid based particles

The particles are liposomes. Liposomes are vesicles comprising at least one lipid bilayer and an internal typically aqueous compartment. Liposomes may be anionic, neutral or cationic. Liposomes may comprise, without limitation, DOPC, DOPG, DOTMA, DOTAP, DOTIM, DDAB, alone or together with cholesterol, to yield DOTMA and cholesterol, DOTAP and cholesterol, DOTIM and cholesterol, and DDAB and cholesterol. In some embodiments, the particles of the invention may be unilamellar liposomal vesicles. In some embodiments, the particles of the invention may be multilamellar liposomal vesicles. In some embodiments, the particles may be interbilayer crosslinked multilamellar vesicles (ICMVs), which are multilamellar lipid vesicles having crosslinked lipid bilayers. Such particles are described in greater detail in U.S. application numbers US 2011/0229529 A1 and US 2012/0177724 A1, each of which is incorporated by reference herein.

### Particle conjugation

In some embodiments, particles comprise antibodies or antibody fragments on their surface. In some embodiments, the particles comprise non-antibody-based ligands on their surface. Non-antibody based ligands include, but are not limited, to cytokines, a term used generically to embrace cytokines, interleukins, and growth factors generally.

In some embodiments, the antibodies are designed to bind to target cells without triggering their elimination by complement or other antibody effector mechanisms. This is achieved either by using antibody fragments or antibodies with mutations that abrogate Fc receptor binding or other effector mechanisms.

These antibody and non-antibody based ligands may be conjugated (or attached or bound, as the terms are used interchangeably herein) to the particle surface covalently or non-covalently. The particles may be synthesized or modified post-synthesis to comprise one or more reactive groups on their exterior surface that can be used to conjugate the antibody and non-antibody based ligands. These particle reactive groups include without limitation thiol-reactive maleimide head groups, haloacetyl (*e.g*., iodoacetyl) groups, imidoester groups, N-hydroxysuccinimide esters, pyridyl disulfide groups, and the like. As an example, particles may be synthesized to include maleimide conjugated phospholipids such as, without limitation, DSPE-MaL-PEG2000. It will be understood that when surface modified in this manner, the particles are intended for use with ligands having "complementary" reactive groups (i.e., reactive groups that react with those of the particles).

Methods for conjugating ligands or receptors such as antibodies to particle surfaces are described by Kwong et al. Cancer Research, 2013, 73:1547-1558, the entire contents of which are incorporated by reference herein.

### Agents

The invention contemplates the delivery of agents to particular cells, and thus potentially to localized regions or tissues *in vivo.* As used herein, an agent is any atom or molecule or compound that can be used to provide benefit to a subject (including without limitation prophylactic or therapeutic benefit). The agents of particular interest, in some embodiments, are those that exert an effect on target cells, whether directly or indirectly. Some agents may exert their effects on tumor cells, pathogens, or pathogen-infected cells. The nature of the agent will depend on the particular application, as should be apparent.

The particles may carry the agent internally including for example in pores or in a hollow core. The particles may carry the agent on its surface. The particles may carry the agent internally and on its surface.

The invention further contemplates that one or more agents may be used alongside of the particles of the invention, although not conjugated to or encapsulated within. For example, the particles of the invention may be formulated together with one or more agents.

The agent may be without limitation a chemical entity, a protein, a polypeptide, a peptide, a nucleic acid, a virus-like particle, a steroid, a proteoglycan, a lipid, a carbohydrate, and analogs, derivatives, mixtures, fusions, combinations or conjugates thereof. The agent may be a pro-drug that is metabolized and thus converted *in vivo* to its active (and/or stable) form.

The agents may be naturally occurring or non-naturally occurring. Naturally occurring agents include those capable of being synthesized by the subjects to whom the particles are administered. Non-naturally occurring are those that do not exist in nature normally, whether produced by plant, animal, microbe or other living organism.

One class of agents that can be delivered in a localized manner using the particles of the invention includes chemical compounds that are non-naturally occurring, or chemical compounds that are not naturally synthesized by mammalian (and in particular human) cells.

A variety of agents that are currently used for therapeutic purposes can be delivered according to the invention and these include without limitation immunomodulatory agents such as immunostimulatory agents, antigens, adjuvants, imaging agents, anti-cancer agents, anti-infective agents, and the like.

One particular class of agents is inhibitors of immunosuppression. Examples include Shp1/2 protein tyrosine phosphatase (PTPase) inhibitor (NSC-87877; CAS 56932-43-5), sunitinib, or other inhibitors of receptor tyrosine kinases, or p38 MAPK inhibitors including MAPK pathway inhibitors.

The p38 MAPK pathway inhibitor may be a RAF inhibitor such as a pan-RAF inhibitor or a selective RAF inhibitor. Examples of RAF inhibitors include RAF265, sorafenib, dabrafenib (GSK2118436), SB590885, PLX 4720, PLX4032, GDC-0879 and ZM 336372.

The p38 MAPK pathway inhibitor may be a MEK inhibitor. Examples of MEK inhibitors include CI-1040/PD184352, AZD6244, PD318088, PD98059, PD334581, RDEA119, 6-Methoxy-7-(3-morpholin-4-yl-propoxy)-4-(4-phenoxy-phenylamino)-quinoline-3-carbonitrile and 4-[3-Chloro-4-(1-methyl-1H-imidazol-2-ylsulfanyl)-phenylamino]-6-methoxy-7-(3-morpholin-4-yl-propoxy)-quinoline-3-carbonitrile, trametinib (GSK1120212), and ARRY-438162.

The p38 MAPK pathway inhibitor may be an ERK inhibitor. Examples of ERK inhibtors include VTX11e, AEZS-131, PD98059, FR180204, and FR148083.

Still other p38 MAPK inhibitors are Tocriset, SB239063, SB203580, pamapimod,, dilmapimod, and PH797804.

*Imaging Agents.* As used herein, an imaging agent is an agent that emits signal directly or indirectly thereby allowing its detection *in vivo.* Imaging agents such as contrast agents and radioactive agents that can be detected using medical imaging techniques such as nuclear medicine scans and magnetic resonance imaging (MRI). Imaging agents for magnetic resonance imaging (MRI) include Gd(DOTA), iron oxide or gold nanoparticles; imaging agents for nuclear medicine include 201T1, gamma-emitting radionuclide 99 mTc; imaging agents for positron-emission tomography (PET) include positron-emitting isotopes, (18)F-fluorodeoxyglucose ((18)FDG), (18)F-fluoride, copper-64, gadoamide, and radioisotopes of Pb(II) such as 203 Pb, and 11In; imaging agents for *in vivo* fluorescence imaging such as fluorescent dyes or dye-conjugated nanoparticles. In other embodiments, the agent to be delivered is conjugated, or fused to, or mixed or combined with an imaging agent.

*Immunostimulatory Agents.* As used herein, an immunostimulatory agent is an agent that stimulates an immune response (including enhancing a pre-existing immune response) in a subject to whom it is administered, whether alone or in combination with another agent. Examples include antigens, adjuvants (*e.g*., TLR ligands such as imiquimod, imidazoquinoline, nucleic acids comprising an unmethylated CpG dinucleotide, monophosphoryl lipid A or other lipopolysaccharide derivatives, single-stranded or double-stranded RNA, flagellin, muramyl dipeptide), cytokines including interleukins (*e.g*., IL-2, IL-7, IL-15 (or superagonist/mutant forms of these cytokines), IL-12, IFN-gamma, IFN-alpha, GM-CSF, FLT3-ligand, etc.), immunostimulatory antibodies (*e.g*., anti-CTLA-4, anti-CD28, anti-CD3, or single chain/antibody fragments of these molecules), and the like.

*Adjuvants.* The adjuvant may be without limitation alum (*e.g.,* aluminum hydroxide, aluminum phosphate); saponins purified from the bark of the *Q. saponaria* tree such as QS21 (a glycolipid that elutes in the 21st peak with HPLC fractionation; Antigenics, Inc., Worcester, Mass.); poly[di(carboxylatophenoxy)phosphazene (PCPP polymer; Virus Research Institute, USA), Flt3 ligand, *Leishmania* elongation factor (a purified *Leishmania* protein; Corixa Corporation, Seattle, Wash.), ISCOMS (immunostimulating complexes which contain mixed saponins, lipids and form virus-sized particles with pores that can hold antigen; CSL, Melbourne, Australia), Pam3Cys, SB-AS4 (SmithKline Beecham adjuvant system #4 which contains alum and MPL; SBB, Belgium), non-ionic block copolymers that form micelles such as CRL 1005 (these contain a linear chain of hydrophobic polyoxypropylene flanked by chains of polyoxyethylene, Vaxcel, Inc., Norcross, Ga.), and Montanide IMS (*e.g*., IMS 1312, water-based nanoparticles combined with a soluble immunostimulant, Seppic)

Adjuvants may be TLR ligands. Adjuvants that act through TLR3 include without limitation double-stranded RNA. Adjuvants that act through TLR4 include without limitation derivatives of lipopolysaccharides such as monophosphoryl lipid A (MPLA; Ribi ImmunoChem Research, Inc., Hamilton, Mont.) and muramyl dipeptide (MDP; Ribi) andthreonyl-muramyl dipeptide (t-MDP; Ribi); OM-174 (a glucosamine disaccharide related to lipid A; OM Pharma SA, Meyrin, Switzerland). Adjuvants that act through TLR5 include without limitation flagellin. Adjuvants that act through TLR7 and/or TLR8 include single-stranded RNA, oligoribonucleotides (ORN), synthetic low molecular weight compounds such as imidazoquinolinamines (*e.g*., imiquimod, resiquimod). Adjuvants acting through TLR9 include DNA of viral or bacterial origin, or synthetic oligodeoxynucleotides (ODN), such as CpG ODN. Another adjuvant class is phosphorothioate containing molecules such as phosphorothioate nucleotide analogs and nucleic acids containing phosphorothioate backbone linkages.

*Immunoinhibitory Agents.* As used herein, an immunoinhibitory agent is an agent that inhibits an immune response in a subject to whom it is administered, whether alone or in combination with another agent. Examples include steroids, retinoic acid, dexamethasone, cyclophosphamide, anti-CD3 antibody or antibody fragment, and other immunosuppressants.

*Anti-Cancer Agents.* As used herein, an anti-cancer agent is an agent that at least partially inhibits the development or progression of a cancer, including inhibiting in whole or in part symptoms associated with the cancer even if only for the short term. Several anti-cancer agents can be categorized as DNA damaging agents and these include topoisomerase inhibitors (*e.g*., etoposide, ramptothecin, topotecan, teniposide, mitoxantrone), DNA alkylating agents (*e.g*., cisplatin, mechlorethamine, cyclophosphamide, ifosfamide, melphalan, chorambucil, busulfan, thiotepa, carmustine, lomustine, carboplatin, dacarbazine, procarbazine), DNA strand break inducing agents (*e.g*., bleomycin, doxorubicin, daunorubicin, idarubicin, mitomycin C), anti-microtubule agents (*e.g*., vincristine, vinblastine), anti-metabolic agents (*e.g*., cytarabine, methotrexate, hydroxyurea, 5-fluorouracil, floxuridine, 6-thioguanine, 6-mercaptopurine, fludarabine, pentostatin, chlorodeoxyadenosine), anthracyclines, vinca alkaloids. or epipodophyllotoxins.

Examples of anti-cancer agents include without limitation Acivicin; Aclarubicin; Acodazole Hydrochloride; Acronine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Bortezomib (VELCADE); Brequinar Sodium; Bropirimine; Busulfan; Cactinomycin; Calusterone; Caracemide; Carbetimer; Carboplatin (a platinum-containing regimen); Carmustine; Carubicin Hydrochloride; Carzelesin; Cedefingol; Chlorambucil; Cirolemycin; Cisplatin (a platinum-containing regimen); Cladribine; Crisnatol Mesylate; Cyclophosphamide; Cytarabine; Dacarbazine; Dactinomycin; Daunorubicin; Decitabine; Dexormaplatin; Dezaguanine; Diaziquone; Docetaxel (TAXOTERE); Doxorubicin; Droloxifene; Dromostanolone; Duazomycin; Edatrexate; Eflornithine; Elsamitrucin; Enloplatin; Enpromate; Epipropidine; Epirubicin; Erbulozole; Erlotinib (TARCEVA), Esorubicin; Estramustine; Etanidazole; Etoposide; Etoprine; Fadrozole; Fazarabine; Fenretinide; Floxuridine; Fludarabine; 5-Fluorouracil; Flurocitabine; Fosquidone; Fostriecin; Gefitinib (IRESSA), Gemcitabine; Hydroxyurea; Idarubicin; Ifosfamide; Ilmofosine; Imatinib mesylate (GLEEVAC); Interferon alpha-2a; Interferon alpha-2b; Interferon alpha-nl; Interferon alpha-n3; Interferon beta-I a; Interferon gamma-I b; Iproplatin; Irinotecan; Lanreotide; Lenalidomide (REVLIMID, REVIMID); Letrozole; Leuprolide; Liarozole; Lometrexol; Lomustine; Losoxantrone; Masoprocol; Maytansine; Mechlorethamine; Megestrol; Melengestrol; Melphalan; Menogaril; Mercaptopurine; Methotrexate; Metoprine; Meturedepa; Mitindomide; Mitocarcin; Mitocromin; Mitogillin; Mitomalcin; Mitomycin; Mitosper; Mitotane; Mitoxantrone; Mycophenolic Acid; Nocodazole; Nogalamycin; Ormaplatin; Oxisuran; Paclitaxel; Pemetrexed (ALIMTA), Pegaspargase; Peliomycin; Pentamustine; Pentomone; Peplomycin; Perfosfamide; Pipobroman; Piposulfan; Piritrexim Isethionate; Piroxantrone; Plicamycin; Plomestane; Porfimer; Porfiromycin; Prednimustine; Procarbazine; Puromycin; Pyrazofurin; Riboprine; Rogletimide; Safingol; Semustine; Simtrazene; Sitogluside; Sparfosate; Sparsomycin; Spirogermanium; Spiromustine; Spiroplatin; Streptonigrin; Streptozocin; Sulofenur; Talisomycin; Tamsulosin; Taxol; Taxotere; Tecogalan; Tegafur; Teloxantrone; Temoporfin; Temozolomide (TEMODAR); Teniposide; Teroxirone; Testolactone; Thalidomide (THALOMID) and derivatives thereof; Thiamiprine; Thioguanine; Thiotepa; Tiazofurin; Tirapazamine; Topotecan; Toremifene; Trestolone; Triciribine; Trimetrexate; Triptorelin; Tubulozole; Uracil Mustard; Uredepa; Vapreotide; Verteporfin; Vinblastine; Vincristine; Vindesine; Vinepidine; Vinglycinate; Vinleurosine; Vinorelbine; Vinrosidine; Vinzolidine; Vorozole; Zeniplatin; Zinostatin; Zorubicin.

The anti-cancer agent may be an enzyme inhibitor including without limitation tyrosine kinase inhibitor, a CDK inhibitor, a MAP kinase inhibitor, or an EGFR inhibitor. The tyrosine kinase inhibitor may be without limitation Genistein (4',5,7-trihydroxyisoflavone), Tyrphostin 25 (3,4,5-trihydroxyphenyl), methylene]-propanedinitrile, Herbimycin A, Daidzein (4',7-dihydroxyisoflavone), AG-126, trans-1-(3'-carboxy-4'-hydroxyphenyl)-2-(2",5"-dihydroxyphenyl)ethane, or HDBA (2-Hydroxy5-(2,5-Dihydroxybenzylamino)-2-hydroxybenzoic acid. The CDK inhibitor may be without limitation p21, p27, p57, p15, p16, p18, or p19. The MAP kinase inhibitor may be without limitation KY12420 (C₂₃H₂₄O₈), CNI-1493, PD98059, or 4-(4-Fluorophenyl)-2-(4-methylsulfinyl phenyl)-5-(4-pyridyl) 1H-imidazole. The EGFR inhibitor may be without limitation erlotinib (TARCEVA), gefitinib (IRESSA), WHI-P97 (quinazoline derivative), LFM-A12 (leflunomide metabolite analog), ABX-EGF, lapatinib, canertinib, ZD-6474 (ZACTIMA), AEE788, and AG1458.

The anti-cancer agent may be a VEGF inhibitor including without limitation bevacizumab (AVASTIN), ranibizumab (LUCENTIS), pegaptanib (MACUGEN), sorafenib, sunitinib (SUTENT), vatalanib, ZD-6474 (ZACTIMA), anecortave (RETAANE), squalamine lactate, and semaphorin.

The anti-cancer agent may be an antibody or an antibody fragment including without limitation an antibody or an antibody fragment including but not limited to bevacizumab (AVASTIN), trastuzumab (HERCEPTIN), alemtuzumab (CAMPATH, indicated for B cell chronic lymphocytic leukemia,), gemtuzumab (MYLOTARG, hP67.6, anti-CD33, indicated for leukemia such as acute myeloid leukemia), rituximab (RITUXAN), tositumomab (BEXXAR, anti-CD20, indicated for B cell malignancy), MDX-210 (bispecific antibody that binds simultaneously to HER-2/neu oncogene protein product and type I Fc receptors for immunoglobulin G (IgG) (Fc gamma RI)), oregovomab (OVAREX, indicated for ovarian cancer), edrecolomab (PANOREX), daclizumab (ZENAPAX), palivizumab (SYNAGIS, indicated for respiratory conditions such as RSV infection), ibritumomab tiuxetan (ZEVALIN, indicated for Non-Hodgkin's lymphoma), cetuximab (ERBITUX), MDX-447, MDX-22, MDX-220 (anti-TAG-72), IOR-C5, IOR-T6 (anti-CD1), IOR EGF/R3, celogovab (ONCOSCINT OV103), epratuzumab (LYMPHOCIDE), pemtumomab (THERAGYN), and Gliomab-H (indicated for brain cancer, melanoma).

*Anti-Infective Agents*. The agent may be an anti-infective agent including without limitation an anti-bacterial agent, an anti-viral agent, an anti-parasitic agent, an anti-fungal agent, and an anti-mycobacterial agent.

Anti-bacterial agents may be without limitation β-lactam antibiotics, penicillins (such as natural penicillins, aminopenicillins, penicillinase-resistant penicillins, carboxy penicillins, ureido penicillins), cephalosporins (first generation, second generation, and third generation cephalosporins), other β-lactams (such as imipenem, monobactams), β-lactamase inhibitors, vancomycin, aminoglycosides and spectinomycin, tetracyclines, chloramphenicol, erythromycin, lincomycin, clindamycin, rifampin, metronidazole, polymyxins, sulfonamides and trimethoprim, or quinolines.

Other anti-bacterials may be without limitation Acedapsone; Acetosulfone Sodium; Alamecin; Alexidine; Amdinocillin; Amdinocillin Pivoxil; Amicycline; Amifloxacin; Amifloxacin Mesylate; Amikacin; Amikacin Sulfate; Aminosalicylic acid; Aminosalicylate sodium; Amoxicillin; Amphomycin; Ampicillin; Ampicillin Sodium; Apalcillin Sodium; Apramycin; Aspartocin; Astromicin Sulfate; Avilamycin; Avoparcin; Azithromycin; Azlocillin; Azlocillin Sodium; Bacampicillin Hydrochloride; Bacitracin; Bacitracin Methylene Disalicylate; Bacitracin Zinc; Bambermycins; Benzoylpas Calcium; Berythromycin; Betamicin Sulfate; Biapenem; Biniramycin; Biphenamine Hydrochloride; Bispyrithione Magsulfex; Butikacin; Butirosin Sulfate; Capreomycin Sulfate; Carbadox; Carbenicillin Disodium; Carbenicillin Indanyl Sodium; Carbenicillin Phenyl Sodium; Carbenicillin Potassium; Carumonam Sodium; Cefaclor; Cefadroxil; Cefamandole; Cefamandole Nafate; Cefamandole Sodium; Cefaparole; Cefatrizine; Cefazaflur Sodium; Cefazolin; Cefazolin Sodium; Cefbuperazone; Cefdinir; Cefepime; Cefepime Hydrochloride; Cefetecol; Cefixime; Cefmenoxime Hydrochloride; Cefmetazole; Cefmetazole Sodium; Cefonicid Monosodium; Cefonicid Sodium; Cefoperazone Sodium; Ceforanide; Cefotaxime Sodium; Cefotetan; Cefotetan Disodium; Cefotiam Hydrochloride; Cefoxitin; Cefoxitin Sodium; Cefpimizole; Cefpimizole Sodium; Cefpiramide; Cefpiramide Sodium; Cefpirome Sulfate; Cefpodoxime Proxetil; Cefprozil; Cefroxadine; Cefsulodin Sodium; Ceftazidime; Ceftibuten; Ceftizoxime Sodium; Ceftriaxone Sodium; Cefuroxime; Cefuroxime Axetil; Cefuroxime Pivoxetil; Cefuroxime Sodium; Cephacetrile Sodium; Cephalexin; Cephalexin Hydrochloride; Cephaloglycin; Cephaloridine; Cephalothin Sodium; Cephapirin Sodium; Cephradine; Cetocycline Hydrochloride; Cetophenicol; Chloramphenicol; Chloramphenicol Palmitate; Chloramphenicol Pantothenate Complex; Chloramphenicol Sodium Succinate; Chlorhexidine Phosphanilate; Chloroxylenol; Chlortetracycline Bisulfate; Chlortetracycline Hydrochloride; Cinoxacin; Ciprofloxacin; Ciprofloxacin Hydrochloride; Cirolemycin; Clarithromycin; Clinafloxacin Hydrochloride; Clindamycin; Clindamycin Hydrochloride; Clindamycin Palmitate Hydrochloride; Clindamycin Phosphate; Clofazimine; Cloxacillin Benzathine; Cloxacillin Sodium; Cloxyquin; Colistimethate Sodium; Colistin Sulfate; Coumermycin; Coumermycin Sodium; Cyclacillin; Cycloserine; Dalfopristin; Dapsone; Daptomycin; Demeclocycline; Demeclocycline Hydrochloride; Demecycline; Denofungin; Diaveridine; Dicloxacillin; Dicloxacillin Sodium; Dihydrostreptomycin Sulfate; Dipyrithione; Dirithromycin; Doxycycline; Doxycycline Calcium; Doxycycline Fosfatex; Doxycycline Hyclate; Droxacin Sodium; Enoxacin; Epicillin; Epitetracycline Hydrochloride; Erythromycin; Erythromycin Acistrate; Erythromycin Estolate; Erythromycin Ethylsuccinate; Erythromycin Gluceptate; Erythromycin Lactobionate; Erythromycin Propionate; Erythromycin Stearate; Ethambutol Hydrochloride; Ethionamide; Fleroxacin; Floxacillin; Fludalanine; Flumequine; Fosfomycin; Fosfomycin Tromethamine; Fumoxicillin; Furazolium Chloride; Furazolium Tartrate; Fusidate Sodium; Fusidic Acid; Gentamicin Sulfate; Gloximonam; Gramicidin; Haloprogin; Hetacillin; Hetacillin Potassium; Hexedine; Ibafloxacin; Imipenem; Isoconazole; Isepamicin; Isoniazid; Josamycin; Kanamycin Sulfate; Kitasamycin; Levofuraltadone; Levopropylcillin Potassium; Lexithromycin; Lincomycin; Lincomycin Hydrochloride; Lomefloxacin; Lomefloxacin Hydrochloride; Lomefloxacin Mesylate; Loracarbef; Mafenide; Meclocycline; Meclocycline Sulfosalicylate; Megalomicin Potassium Phosphate; Mequidox; Meropenem; Methacycline; Methacycline Hydrochloride; Methenamine; Methenamine Hippurate; Methenamine Mandelate; Methicillin Sodium; Metioprim; Metronidazole Hydrochloride; Metronidazole Phosphate; Mezlocillin; Mezlocillin Sodium; Minocycline; Minocycline Hydrochloride; Mirincamycin Hydrochloride; Monensin; Monensin Sodium; Nafcillin Sodium; Nalidixate Sodium; Nalidixic Acid; Natamycin; Nebramycin; Neomycin Palmitate; Neomycin Sulfate; Neomycin Undecylenate; Netilmicin Sulfate; Neutramycin; Nifuradene; Nifuraldezone; Nifuratel; Nifuratrone; Nifurdazil; Nifurimide; Nifurpirinol; Nifurquinazol; Nifurthiazole; Nitrocycline; Nitrofurantoin; Nitromide; Norfloxacin; Novobiocin Sodium; Ofloxacin; Ormetoprim; Oxacillin Sodium; Oximonam; Oximonam Sodium; Oxolinic Acid; Oxytetracycline; Oxytetracycline Calcium; Oxytetracycline Hydrochloride; Paldimycin; Parachlorophenol; Paulomycin; Pefloxacin; Pefloxacin Mesylate; Penamecillin; Penicillin G Benzathine; Penicillin G Potassium; Penicillin G Procaine; Penicillin G Sodium; Penicillin V; Penicillin V Benzathine; Penicillin V Hydrabamine; Penicillin V Potassium; Pentizidone Sodium; Phenyl Aminosalicylate; Piperacillin Sodium; Pirbenicillin Sodium; Piridicillin Sodium; Pirlimycin Hydrochloride; Pivampicillin Hydrochloride; Pivampicillin Pamoate; Pivampicillin Probenate; Polymyxin B Sulfate; Porfiromycin; Propikacin; Pyrazinamide; Pyrithione Zinc; Quindecamine Acetate; Quinupristin; Racephenicol; Ramoplanin; Ranimycin; Relomycin; Repromicin; Rifabutin; Rifametane; Rifamexil; Rifamide; Rifampin; Rifapentine; Rifaximin; Rolitetracycline; Rolitetracycline Nitrate; Rosaramicin; Rosaramicin Butyrate; Rosaramicin Propionate; Rosaramicin Sodium Phosphate; Rosaramicin Stearate; Rosoxacin; Roxarsone; Roxithromycin; Sancycline; Sanfetrinem Sodium; Sarmoxicillin; Sarpicillin; Scopafungin; Sisomicin; Sisomicin Sulfate; Sparfloxacin; Spectinomycin Hydrochloride; Spiramycin; Stallimycin Hydrochloride; Steffimycin; Streptomycin Sulfate; Streptonicozid; Sulfabenz; Sulfabenzamide; Sulfacetamide; Sulfacetamide Sodium; Sulfacytine; Sulfadiazine; Sulfadiazine Sodium; Sulfadoxine; Sulfalene; Sulfamerazine; Sulfameter; Sulfamethazine; Sulfamethizole; Sulfamethoxazole; Sulfamonomethoxine; Sulfamoxole; Sulfanilate Zinc; Sulfanitran; Sulfasalazine; Sulfasomizole; Sulfathiazole; Sulfazamet; Sulfisoxazole; Sulfisoxazole Acetyl; Sulfisoxazole Diolamine; Sulfomyxin; Sulopenem; Sultamicillin; Suncillin Sodium; Talampicillin Hydrochloride; Teicoplanin; Temafloxacin Hydrochloride; Temocillin; Tetracycline; Tetracycline Hydrochloride; Tetracycline Phosphate Complex; Tetroxoprim; Thiamphenicol; Thiphencillin Potassium; Ticarcillin Cresyl Sodium; Ticarcillin Disodium; Ticarcillin Monosodium; Ticlatone; Tiodonium Chloride; Tobramycin; Tobramycin Sulfate; Tosufloxacin; Trimethoprim; Trimethoprim Sulfate; Trisulfapyrimidines; Troleandomycin; Trospectomycin Sulfate; Tyrothricin; Vancomycin; Vancomycin Hydrochloride; Virginiamycin; or Zorbamycin.

Anti-mycobacterial agents may be without limitation Myambutol (Ethambutol Hydrochloride), Dapsone (4,4'-diaminodiphenylsulfone), Paser Granules (aminosalicylic acid granules), Priftin (rifapentine), Pyrazinamide, Isoniazid, Rifadin (Rifampin), Rifadin IV, Rifamate (Rifampin and Isoniazid), Rifater (Rifampin, Isoniazid, and Pyrazinamide), Streptomycin Sulfate or Trecator-SC (Ethionamide).

Anti-viral agents may be without limitation amantidine and rimantadine, ribivarin, acyclovir, vidarabine, trifluorothymidine, ganciclovir, zidovudine, retinovir, and interferons.

Anti-viral agents may be without limitation further include Acemannan; Acyclovir; Acyclovir Sodium; Adefovir; Alovudine; Alvircept Sudotox; Amantadine Hydrochloride; Aranotin; Arildone; Atevirdine Mesylate; Avridine; Cidofovir; Cipamfylline; Cytarabine Hydrochloride; Delavirdine Mesylate; Desciclovir; Didanosine; Disoxaril; Edoxudine; Enviradene; Enviroxime; Famciclovir; Famotine Hydrochloride; Fiacitabine; Fialuridine; Fosarilate; Foscarnet Sodium; Fosfonet Sodium; Ganciclovir; Ganciclovir Sodium; Idoxuridine; Kethoxal; Lamivudine; Lobucavir; Memotine Hydrochloride; Methisazone; Nevirapine; Penciclovir; Pirodavir; Ribavirin; Rimantadine Hydrochloride; Saquinavir Mesylate; Somantadine Hydrochloride; Sorivudine; Statolon; Stavudine; Tilorone Hydrochloride; Trifluridine; Valacyclovir Hydrochloride; Vidarabine; Vidarabine Phosphate; Vidarabine Sodium Phosphate; Viroxime; Zalcitabine; Zidovudine; Zinviroxime or integrase inhibitors.

Anti-fungal agents may be without limitation imidazoles and triazoles, polyene macrolide antibiotics, griseofulvin, amphotericin B, and flucytosine. Antiparasites include heavy metals, antimalarial quinolines, folate antagonists, nitroimidazoles, benzimidazoles, avermectins, praxiquantel, ornithine decarboxylase inhbitors, phenols (*e.g*., bithionol, niclosamide); synthetic alkaloid (*e.g*., dehydroemetine); piperazines (*e.g*., diethylcarbamazine); acetanilide (*e.g.,* diloxanide furonate); halogenated quinolines (*e.g*., iodoquinol (diiodohydroxyquin)); nitrofurans (*e.g*., nifurtimox); diamidines (*e.g*., pentamidine); tetrahydropyrimidine (*e.g*., pyrantel pamoate); or sulfated naphthylamine (*e.g*., suramin).

Other anti-infective agents may be without limitation Difloxacin Hydrochloride; Lauryl Isoquinolinium Bromide; Moxalactam Disodium; Ornidazole; Pentisomicin; Sarafloxacin Hydrochloride; Protease inhibitors of HIV and other retroviruses; Integrase Inhibitors of HIV and other retroviruses; Cefaclor (Ceclor); Acyclovir (Zovirax); Norfloxacin (Noroxin); Cefoxitin (Mefoxin); Cefuroxime axetil (Ceftin); Ciprofloxacin (Cipro); Aminacrine Hydrochloride; Benzethonium Chloride : Bithionolate Sodium; Bromchlorenone; Carbamide Peroxide; Cetalkonium Chloride; Cetylpyridinium Chloride : Chlorhexidine Hydrochloride; Clioquinol; Domiphen Bromide; Fenticlor; Fludazonium Chloride; Fuchsin, Basic; Furazolidone; Gentian Violet; Halquinols; Hexachlorophene : Hydrogen Peroxide; Ichthammol; Imidecyl Iodine; Iodine; Isopropyl Alcohol; Mafenide Acetate; Meralein Sodium; Mercufenol Chloride; Mercury, Ammoniated; Methylbenzethonium Chloride; Nitrofurazone; Nitromersol; Octenidine Hydrochloride; Oxychlorosene; Oxychlorosene Sodium; Parachlorophenol, Camphorated; Potassium Permanganate; Povidone-Iodine; Sepazonium Chloride; Silver Nitrate; Sulfadiazine, Silver; Symclosene; Thimerfonate Sodium; Thimerosal; or Troclosene Potassium.

### Subjects

The invention can be practiced in virtually any subject type. Human subjects are preferred subjects in some embodiments of the invention. Subjects also include animals such as household pets (*e.g.,* dogs, cats, rabbits, ferrets, etc.), livestock or farm animals (*e.g*., cows, pigs, sheep, chickens and other poultry), horses such as thoroughbred horses, laboratory animals (*e.g*., mice, rats, rabbits, etc.), and the like. Subjects also include fish and other aquatic species.

The subjects may have or may be at risk of developing a condition that can benefit from the methods of the invention. Such conditions include cancer (*e.g*., solid tumor cancers), infections, autoimmune disorders, allergies or allergic conditions, asthma, transplant rejection, and the like.

The subject may be undergoing adoptive cell therapy. Such a subject may have already received adoptive cell therapy, or may be receiving adoptive cell therapy, or will receive adoptive cell therapy in the near future. The adoptive cell therapy may take the form of tumor-reactive T cells.

Tests for diagnosing various of the conditions embraced by the invention are known in the art and will be familiar to the ordinary medical practitioner. These laboratory tests include without limitation microscopic analyses, cultivation dependent tests (such as cultures), and nucleic acid detection tests. These include wet mounts, stain-enhanced microscopy, immune microscopy (*e.g*., FISH), hybridization microscopy, particle agglutination, enzyme-linked immunosorbent assays, urine screening tests, DNA probe hybridization, serologic tests, etc. The medical practitioner will generally also take a full history and conduct a complete physical examination in addition to running the laboratory tests listed above.

A subject having a cancer is a subject that has detectable cancer cells. A subject at risk of developing a cancer is a subject that has a higher than normal probability of developing cancer. These subjects include, for instance, subjects having a genetic abnormality that has been demonstrated to be associated with a higher likelihood of developing a cancer, subjects having a familial disposition to cancer, subjects exposed to cancer causing agents (i.e., carcinogens) such as tobacco, asbestos, or other chemical toxins, and subjects previously treated for cancer and in apparent remission.

Subjects having an infection are those that exhibit symptoms thereof including without limitation fever, chills, myalgia, photophobia, pharyngitis, acute lymphadenopathy, splenomegaly, gastrointestinal upset, leukocytosis or leukopenia, and/or those in whom infectious pathogens or byproducts thereof can be detected.

A subject at risk of developing an infection is one that is at risk of exposure to an infectious pathogen. Such subjects include those that live in an area where such pathogens are known to exist and where such infections are common. These subjects also include those that engage in high risk activities such as sharing of needles, engaging in unprotected sexual activity, routine contact with infected samples of subjects (*e.g*., medical practitioners), people who have undergone surgery, including but not limited to abdominal surgery, etc.

The subject may have or may be at risk of developing an infection such as a bacterial infection, a viral infection, a fungal infection, a parasitic infection or a mycobacterial infection. In these embodiments, the particles may comprise an anti-microbial agent such as an anti-bacterial agent, an anti-viral agent, an anti-fungal agent, an anti-parasitic agent, or an anti-mycobacterial agent.

### Cancer

The invention contemplates administration of the particles of the invention to subjects having or at risk of developing a cancer including for example a solid tumor cancer. The cancer may be carcinoma, sarcoma or melanoma. Carcinomas include without limitation to basal cell carcinoma, biliary tract cancer, bladder cancer, breast cancer, cervical cancer, choriocarcinoma, CNS cancer, colon and rectum cancer, kidney or renal cell cancer, larynx cancer, liver cancer, small cell lung cancer, non-small cell lung cancer (NSCLC, including adenocarcinoma, giant (or oat) cell carcinoma, and squamous cell carcinoma), oral cavity cancer, ovarian cancer, pancreatic cancer, prostate cancer, skin cancer (including basal cell cancer and squamous cell cancer), stomach cancer, testicular cancer, thyroid cancer, uterine cancer, rectal cancer, cancer of the respiratory system, and cancer of the urinary system.

Sarcomas are rare mesenchymal neoplasms that arise in bone (osteosarcomas) and soft tissues (fibrosarcomas). Sarcomas include without limitation liposarcomas (including myxoid liposarcomas and pleiomorphic liposarcomas), leiomyosarcomas, rhabdomyosarcomas, malignant peripheral nerve sheath tumors (also called malignant schwannomas, neurofibrosarcomas, or neurogenic sarcomas), Ewing's tumors (including Ewing's sarcoma of bone, extraskeletal (i.e., not bone) Ewing's sarcoma, and primitive neuroectodermal tumor), synovial sarcoma, angiosarcomas, hemangiosarcomas, lymphangiosarcomas, Kaposi's sarcoma, hemangioendothelioma, desmoid tumor (also called aggressive fibromatosis), dermatofibrosarcoma protuberans (DFSP), malignant fibrous histiocytoma (MFH), hemangiopericytoma, malignant mesenchymoma, alveolar soft-part sarcoma, epithelioid sarcoma, clear cell sarcoma, desmoplastic small cell tumor, gastrointestinal stromal tumor (GIST) (also known as GI stromal sarcoma), and chondrosarcoma.

Melanomas are tumors arising from the melanocytic system of the skin and other organs. Examples of melanoma include without limitation lentigo maligna melanoma, superficial spreading melanoma, nodular melanoma, and acral lentiginous melanoma.

The cancer may be a solid tumor lymphoma. Examples include Hodgkin's lymphoma, Non-Hodgkin's lymphoma, and B cell lymphoma.

The cancer may be without limitation bone cancer, brain cancer, breast cancer, colorectal cancer, connective tissue cancer, cancer of the digestive system, endometrial cancer, esophageal cancer, eye cancer, cancer of the head and neck, gastric cancer, intra-epithelial neoplasm, melanoma neuroblastoma, Non-Hodgkin's lymphoma, non-small cell lung cancer, prostate cancer, retinoblastoma, or rhabdomyosarcoma.

### Infection

The invention contemplates administration of the particles to subjects having or at risk of developing an infection such as a bacterial infection, a viral infection, a fungal infection, a parasitic infection or a mycobacterial infection.

The bacterial infection may be without limitation an E. coli infection, a Staphylococcal infection, a Streptococcal infection, a Pseudomonas infection, Clostridium difficile infection, Legionella infection, Pneumococcus infection, Haemophilus infection, Klebsiella infection, Enterobacter infection, Citrobacter infection, Neisseria infection, Shigella infection, Salmonella infection, Listeria infection, Pasteurella infection, Streptobacillus infection, Spirillum infection, Treponema infection, Actinomyces infection, Borrelia infection, Corynebacterium infection, Nocardia infection, Gardnerella infection, Campylobacter infection, Spirochaeta infection, Proteus infection, Bacteriodes infection, H. pylori infection, or anthrax infection.

The mycobacterial infection may be without limitation tuberculosis or leprosy respectively caused by the M. tuberculosis and M. leprae species.

The viral infection may be without limitation a Herpes simplex virus 1 infection, a Herpes simplex virus 2 infection, cytomegalovirus infection, hepatitis A virus infection, hepatitis B virus infection, hepatitis C virus infection, human papilloma virus infection, Epstein Barr virus infection, rotavirus infection, adenovirus infection, influenza A virus infection, H1N1 (swine flu) infection, respiratory syncytial virus infection, varicella-zoster virus infections, small pox infection, monkey pox infection, SARS infection or avian flu infection.

The fungal infection may be without limitation candidiasis, ringworm, histoplasmosis, blastomycosis, paracoccidioidomycosis, crytococcosis, aspergillosis, chromomycosis, mycetoma infections, pseudallescheriasis, or tinea versicolor infection.

The parasite infection may be without limitation amebiasis, Trypanosoma cruzi infection, Fascioliasis, Leishmaniasis, Plasmodium infections, Onchocerciasis, Paragonimiasis, Trypanosoma brucei infection, Pneumocystis infection, Trichomonas vaginalis infection, Taenia infection, Hymenolepsis infection, Echinococcus infections, Schistosomiasis, neurocysticercosis, Necator americanus infection, or Trichuris trichuria infection.

### Effective Amounts, Regimens, Formulations

The particles provided herein may be administered in effective amounts. An effective amount is a dosage sufficient to provide a medically desirable result. The effective amount will vary with the particular condition being treated, the age and physical condition of the subject being treated, the severity of the condition, the duration of the treatment, the nature of the concurrent or combination therapy (if any), the specific route of administration and like factors within the knowledge and expertise of the health practitioner. It is preferred, generally, that a maximum dose be used (i.e., the highest safe dose according to sound medical judgment).

The invention provides compositions, including pharmaceutical compositions, comprising the particles of the invention. Pharmaceutical compositions are compositions that may comprise the particles of the invention, preferably in a pharmaceutically-acceptable carrier. The term "pharmaceutically-acceptable carrier" means one or more compatible solid or liquid filler, diluents or encapsulating substances which are suitable for administration to a human or other subject contemplated by the invention. The term "carrier" denotes an organic or inorganic ingredient, natural or synthetic, with which the particles are suspended to facilitate administration. Components of the pharmaceutical compositions are commingled in a manner that precludes interaction that would substantially impair their desired pharmaceutical efficiency.

The compositions of the invention may be formulated for parenteral administration by injection, e.g., by bolus injection or continuous infusion. Formulations for injection may be presented in unit dosage form, *e.g*., in ampoules or in multi-dose containers. Pharmaceutical parenteral formulations include aqueous solutions of components. Aqueous injection suspensions may contain substances which increase the viscosity of the suspension, such as sodium carboxymethyl cellulose, sorbitol, or dextran. Alternatively, suspensions may be prepared as oil-based suspensions. Suitable lipophilic solvents or vehicles include fatty oils such as sesame oil, or synthetic fatty acid esters, such as ethyl oleate or triglycerides.

The compositions of the invention may be administered parenterally including intravenously or subcutaneously, although other routes of administration are also contemplated.

### Repeated administration

In some embodiments, particles of the invention may be administered before and/or at the same time as and/or after the administration of adoptive cell therapy. In some instances, the particles of the invention may be administered substantially simultaneously with adoptive cell therapy as well as after adoptive cell therapy. Such a subject may be receiving the particles of the invention substantially simultaneously and within days or weeks of receiving adoptive cell therapy. As used herein, substantially simultaneously means within 6 hours, including within 4 hours, within 2 hours, or within 1 hour. The adoptive cell therapy may take the form of tumor-reactive T cells. In some embodiments, the first administration occurs substantially simultaneously with the administration of adoptive cell therapy. In some embodiments, the first administration occurs after the administration of adoptive cell therapy. The second and subsequent administrations may occur after the administration of adoptive cell therapy.

Repeated administration means that the particles of the invention are administered to the subject at least twice. In some embodiments, targeting particles are administered at least 3 times, at least 4 times, at least 5 times, at least 6 times, at least 7 times, at least 8 times, or more. Repeated administration may occur over the course of a week, 2 weeks, 3 weeks, 4 weeks or longer. Repeated administrations may be regularly or randomly spaced in time. They may be days apart, or weeks apart, or months apart. For example, particles of the invention may be administered every day, every 2 days, every 3 days, every 4 days, every 5 days, every 6 days, every week, every 2 weeks, every 3 weeks, every 4 weeks, *etc.*

In some embodiments, the first administration of particles occurs substantially simultaneously with the administration of adoptive cell therapy, and the second administration of particles occurs 3 days later.

It is to be understood that because the particles of the invention can be used with other immunotherapies, it is intended that any embodiments recited herein in the context of adoptive cell therapy are illustrative of such other therapies, and that such other therapies may be used in place of adoptive cell therapies.

### EXAMPLES

### Example 1

In adoptive cell therapy (ACT), autologous tumor-specific T-cells isolated from cancer patients are activated and expanded ex vivo, then infused back into the individual to eliminate metastatic tumors. A major limitation of this promising approach is the rapid loss of ACT T-cell effector function *in vivo* due to the highly immunosuppressive environment in tumors. Protection of T-cells from immunosuppressive signals can be achieved by systemic administration of supporting adjuvant drugs such as interleukins, chemotherapy, and other immunomodulators, but these adjuvant treatments are often accompanied by serious toxicities and may still fail to optimally stimulate lymphocytes in all tumor and lymphoid compartments. Here we propose a novel strategy to repeatedly stimulate or track ACT T-cells, using cytokines or ACT-cell-specific antibodies as ligands to target PEGylated liposomes to transferred T-cells *in vivo.* Using F(ab')2 fragments against a unique cell surface antigen on ACT cells (Thy1.1) or an engineered interleukin-2 (IL-2) molecule on an Fc framework as targeting ligands, we demonstrate that >95% of ACT cells can be conjugated with liposomes following a single injection *in vivo.* Further, we show that IL-2-conjugated liposomes both target ACT cells and are capable of inducing repeated waves of ACT T-cell proliferation in tumor-bearing mice. These results demonstrate the feasibility of repeated functional targeting of T-cells *in vivo,* which will enable delivery of imaging contrast agents, immunomodulators, or chemotherapy agents in adoptive cell therapy regimens or boosting of endogenous T-cell responses against pathogens or tumors.

### Materials

All lipids and polycarbonate membranes (0.2 µm) for size extrusion were from Avanti Polar Lipids (Alabaster, AL) and used as received. DiD, ACK lysis buffer, Calcium Phosphate Transfection Kit, HEK293 Free Style Cells, Max Efficiency ® DH5αTM Competent cells and Phoenix eco viral packaging cells were obtained from Invitrogen Life Technologies (Grand Island, NY). Anti-thy1.1 (clone 19E12) and mouse IgG2a isotype control antibodies were purchased from BioXCell (West Lebanon, NH). Dithiothreitol (DTT), Fluorescein isothiocyanate (FITC) isomer I, Concanavalin A Type VI (ConA), and Triton X-100 were from Sigma-Aldrich (St. Louis, MO) and used as received. Recombinant IL-2 and IL-7 were purchased from PeproTech (Rocky Hill, NJ). Anti-mouse CD16/32, anti-CD25, anti-CD25-Alexa 488, anti-CD8-PE, anti-Thy1.1, anti-Thy1.1-Percp-Cy5.5 and anti-Thy1.1-FITC were from eBiosceince (San Diego, CA). Anti- mouse vβ13 T-cell Receptor-FITC was purchased from Becton Dickinson (Franklin Lakes, NJ). Protein A agarose column and Amicon Ultra-15 30kDa MWCO Centrifugal Filter Units were from Millipore (Billerica, MA). Polyethylenimine (PEI) was from Polysciences (Warrington, PA). F(ab')2 Preparation Kits, BCA Protein Assay Kits, and Zeba desalting columns were from Pierce Thermo Scientific (Rockford, IL). IL-2 ELISA Kits were obtained from R&D Systems (Minneapolis, MN). Ficoll-Pague Plus was from GE Health Care (Waukesha, WI). EasySep™ Mouse CD8+ T Cell Enrichment Kit was from Stemcell (Vancouver, BC, Canada). Collagenase II and Hank's Balanced Salt Solution were purchased from (Gibco-Invitrogen, Carlsbad, CA). EndoFree Plasmid Maxi Kit was from Qiagen (Valencia, CA). Retronectin Recombinant Human Fibronectin Fragment was from Clontech (Mountain View, CA). D-Luciferin was from Caliper Life Sciences (Hopkinton, MA). B16F10 melanoma cells were from American Type Culture Collection (Manassas, VA).

### Methods

***Preparation of IL-2-Fc and anti-Thy1.1 F(ab')2:*** IL-2-Fc is a bivalent fusion protein of the C-terminus of murine wild type IL-2 linked to a mouse IgG2a backbone [23]. A D265A mutation was introduced in the IgG2a Fc region to minimize interaction of IL-2-Fc with Fc receptors [24]. IL-2-Fc gene was transformed into DH5α cells via heat shock and extracted after clone expansion using an EndoFree Plasmid Maxi Kit following the manufacturer's instructions. HEK293 Freestyle cells were transfected with IL-2-Fc gene/Polyethylenimine (PEI) complexes and grown in roller bottles at 37 °C for a week before harvest. Cells were spun down and secreted IL-2-Fc in the supernatant was purified by gravity flow/elution through Protein A agarose columns and concentrated by using centrifugal filter units (Amicon Ultra-15 30kDa MWCO).

Monoclonal antibodies (Abs) against Thy 1.1 were digested with pepsin to generate the F(ab')2 using a F(ab')2 Preparation Kit following the manufacturer's instructions. IL-2-Fc and anti-Thy1.1 F(ab')2 concentrations were determined by the BCA Protein Assay Kit. IL-2-Fc bioactivity concentration relative to wild type murine IL-2 was quantified by an IL-2 ELISA Kit.

***Synthesis of IL-2-Fc-Liposome and anti-Thy1.1-Liposome:*** Vacuum dried lipid films composed of 1,2-distearoyl-sn-glycero-3-phosphoethanolamine-N-[maleimide(polyethylene glycol)-2000 (maleimide-PEG2000-DSPE)/ cholesterol /hydrogenated Soy L-α-phosphatidylcholine (HSPC) in a molar ratio of 2.5/27.5/69 together with 1% of a fluorescent lipophilic tracer dye 1,1'-Dioctadecyl-3,3,3',3'-Tetramethylindodicarbocyanine, 4-Chlorobenzenesulfonate Salt (DiD) were rehydrated in 250 µl of 50 mM HEPES/150 mM NaCl-buffer (pH6.5). Lipids were vortexed every 10 min for 1 hr at 62°C to form vesicles and size extruded through a polycarbonate membrane (0.2 µm). After washing in excess phosphate buffered saline (PBS) pH7.4 and spinning down by ultracentrifugation at 110,000xg for 4 hr, liposomes were re-suspended in 100 µl PBS per 1.4 mg of lipids.

IL-2-Fc and anti-Thy1.1 F(ab')2 were coupled to liposomes as previously described [23]. Briefly, Ab or cytokine (4-8 mg/ml) were treated with 1.8 mM DTT in the presence of 10 mM EDTA at 25°C for 20 min to expose hinge region free thiols. DTT was subsequently removed by using Zeba desalting columns before mixing with maleimide-bearing liposomes (1 mg protein/1 mg lipid) in PBS pH 7.4. After incubation for 18 hr at 25°C on a rotator, excess protein was removed by ultracentrifugation in excess PBS (if aggregation occurs, liposomes were size extruded through a 0.2 µm polycarbonate membrane at 37°C before ultracentrifugation). Liposome sizes were characterized before/after coupling by dynamic light scattering (90Plus Particle Size Analyzer, Brookhaven, Holtsville, NY).

***Quantification of targeting ligands coupled to liposomes**:* Anti-Thy1.1-FITC was concentrated to 4-8 mg/ml using Ultra-15 Centrifugal Filters before coupling to liposomes as previously described. After liposomes were solubilized in 2% Triton X-100 at 37°C for 5 min with gentle vortexing, FITC fluorescence was measured at ex/em wavelengths of 490/520nm using a fluorescence plate reader (Tecan Systems, San Jose, CA) and converted to protein concentrations using standard curves prepared from serial dilutions of neat anti-Thy 1.1-FITC stock solutions. IL-2-Fc-Lips were solubilized in the same manner and the amount of IL-2 coupled was determined using an IL-2 ELISA Kit (R&D Systems, Minneapolis, MN) following the manufacturer's instructions.

***Activation of pmel-1 Thy1.1+CD8+ T-cells:*** Animals were cared for in the USDA-inspected MIT Animal Facility under federal, state, local and NIH guidelines for animal care. Spleens from pmel-1 Thy1.1+ mice were ground through a 70 µm cell strainer and red blood cells were removed by incubating with ACK lysis buffer (2 ml per spleen) for 5 min at 25°C. After 1 wash in PBS, the remaining cells were cultured at 37°C in RPMI 1640 medium containing 10% fetal calf serum (FCS). ConA at a final concentration of 2 µg/ml and IL-7 at 1 ng/ml were added to activate and expand splenocytes. After two days, dead cells were removed by Ficoll-Pague Plus gradient separation and CD8+ T-cells were isolated via magnetic negative selection using an EasySep™ Mouse CD8+ T Cell Enrichment Kit. Purified CD8+ T-cells were re-suspended at 1.5×10⁶ per ml RPMI containing 10 ng/ml recombinant murine IL-2. After 24 hr, cells were washed 3 times in PBS and re-suspended in 100×10⁶ per ml for adoptive transfer.

For bioluminescence imaging experiments, Click Beetle Red luciferase (CBR-luc) [16] was introduced into pmel-1 T-cells (post ficoll purification and magnetic selection) by retroviral transduction. Phoenix eco viral packaging cells were seeded at 4×10⁶ cells per 10 cm tissue culture dish in 10ml DMEM medium containing 10% FCS. After incubation overnight at 37°C, phoenix cells were exchanged with 10ml fresh DMEM with 10% FCS, transfected with CBR-luc plasmid and phoenix eco plasmid using a Calcium Phosphate Transfection Kit and cultured at 32°C for 24 hr. DMEM was then replaced with 6 ml RPMI containing 10% FCS and transfected phoenix eco cells were incubated for another 24 hr. Supernatant containing the retrovirus-packaged CBR-luc gene was collected and replaced with fresh RPMI for another 24 hr incubation. Supernatant was collected again and combined with that collected 24 hr earlier, and sterile filtered (0.45 µm). Six-well non-tissue culture plates (BD Falcon) were coated with 1 ml RetroNectin (15 µg/ml) 18 hr at 4°C, then excess RetroNectin was aspirated. Pmel-1 T-cells post ficoll purification and magnetic selection were suspended in filtered viral sups (RPMI collected previously) with 10 ng/ml IL-2 at 1.8x10⁶/ml, 3 ml was added to each RetroNection-coated well, and spinoculation was conducted by centrifuging at 2000x g for 1 hr at 25°C. Transduced T-cells were then incubated at 37°C. Six hours later, 1 ml of fresh RPMI was added with 10 ng/ml IL-2. Transduced, activated pmel-1 T-cells were used 1 day later for adoptive transfer studies.

***In vitro liposome binding to T-cells:*** DiD-labeled protein-conjugated liposomes (0.7 mg lipids in 100 µl) were incubated with 15×10⁶ activated pmel-1 Thy1.1+ T-cells in 1ml complete RPMI supplemented with 10% FCS for 30 min at 37°C with gentle agitation every 10 min. In competitive conjugation assays, 100-fold molar excess soluble IL-2-Fc or anti-Thy1.1 free antibody (compare to the amount coupled to liposomes) was added 30 min before targeting liposomes to saturate IL-2 or Thy1.1 receptors on the cells, respectively. For IL-2-Fc-Liposome (IL-2-Fc-Lip) competition assays, 2.5x10⁶ activated pmel-1 CD8+ T-cells were mixed with 2.5x10⁶ naive C57B1/6 splenocytes in 100 µl complete RPMI with 10% FCS. The cell mixture was incubated with or without 0.24 mg/ml soluble IL-2-Fc, followed by incubation with 0.07 mg/ml IL-2-Fc-Lip for 30 minutes at 37°C with total volume topped up to 300 µl. For competition assays with anti-Thy1.1.-Liposome (anti-Thy1.1-Lip), 0.15 mg/ml liposomes (Lip) were incubated with a mixture of 2.5 x10⁶ activated pmel-1 T-cells and 2.5 x10⁶ naive C57B1/6 splenocytes (with or without pre-blocking by 1.34 mg/ml anti-Thy1.1). Cells without any liposomes added served as a control for cellular autofluorescence and cells conjugated with 0.15 mg/ml IgG2a-Liposomes (IgG2a-Lip) were used to test non-specific binding of non-targeting liposomes. For all *in vitro* conjugation experiments, cells were stained with anti-CD8 and anti-Thy1.1 after two washes in ice cold PBS to remove unbound liposomes, and analyzed by flow cytometry on a BD FACS Canto except competition assays which were done on a BD LSR II.

***Titration of liposome concentration for in vitro conjugation:*** Varying amounts of DiD-labeled anti-Thy1.1-Lip were added to 5 ×10⁶ activated pmel-1 Thy1.1+ T-cells in 100 µl complete RPMI with 10% FCS. The total volume for all groups was topped up with RPMI with 10% FCS to 300 µl and incubated at 37°C for 30 min. After two washes in ice cold PBS to remove unbound liposomes, cells were resuspended in FACS buffer, surface stained with fluorescently labeled anti-CD8 and anti-Thy1.1, and analyzed by flow cytometry on a BD LSR II.

***Internalization of liposomes:*** Anti-Thy1.1-Liposomes were labeled with 1% (mol) carboxyfluorescein- 1,2-dioleoyl-sn-glycero-3-phosphoethanolamine lipid (CF-DOPE) instead of DiD during the synthesis stage and incubated with 6×10⁶ activated pmel-1 Thy1.1+ T-cells per 0.7 mg of lipids for 60 min at 4°C with gentle agitation every 15 min. After two washes in ice cold PBS pH7.4 to remove unbound liposomes, T-cells were resuspended in RPMI and aliquotted into four equal portions for 0, 2, 4 and 6 hr time points, respectively. After each incubation interval at 37°C, T-cells were washed 2x in ice cold PBS and re-suspended in flow cytometry buffer (2% FCS in PBS). Cells were placed on ice to minimize further internalization and analyzed by flow cytometry on a BD LSR II. Cells were also imaged directly without fixation on a Zeiss LSM 510 laser scanning confocal microscope.

***Adoptive transfer and in vivo liposome targeting:*** Albino C57BL/6 female mice 6-8 weeks of age were from the Jackson Laboratory (Bar Harbor, ME). One day before adoptive transfer, mice were sublethally lymphodepleted with 5Gy total body irradiation. 15×10⁶ activated pmel-1 CD8+ T-cells in 150 µl PBS were injected intravenously (i.v.) into each recipient animal. DiD-labeled immunoliposomes (1.4 mg lipids) were re-suspended in 100 µl PBS and injected i.v. immediately or three days after adoptive transfer. Twenty-four hr after administration of liposomes, mice were euthanized and blood, lymph node, and spleen cells were analyzed by flow cytometry on a BD FACS Canto to assess liposome binding to T-cells.

***Adoptive transfer of CBR-Luc T-cells and bioluminescence imaging:*** B16F10 melanoma cells were suspended at 1×10⁶ cells per 200 µl in Hank's Balanced Salt Solution and injected i.v. to induce lung metastases in albino C57B1/6 mice (Day -8). Animals were then sublethally lymphodepleted by total body irradiation (5 Gy) 7 days post tumor inoculation (Day - 1). Pmel-1 CD8+ T-cells transduced with CBR-Luc (12×10⁶) were resuspended in 150 µl PBS and administered i.v. one day after lymphodepletion (Day 0). IL-2-Fc-Lip (1 mg of liposomes) or PBS were injected i.v. immediately after ACT and on day 6. D-Luciferin, a substrate for CBR-luc, was suspended in PBS (15 mg/ml) and 150 mg luciferin/kg body weight was injected Intraperitoneally (i.p.) in anesthetized animals 10 min before bioluminescence imaging acquisitions (5 min, 3 min, 2min and 1min) on a Xenogen IVIS Spectrum Imaging System (Caliper Life Sciences). Images were collected every two days starting from day 0 (2hrs after ACT) to day 14. Living Image software version 3.0 (Caliper Life Sciences) was used to acquire and quantitate the bioluminescence imaging data sets. To compare the stimulatory effects of soluble IL-2 and IL-2-Fc-Lip, a similar experiment was repeated with 1st/2nd dose as 0.5 mg/1 mg IL-2-Fc-Lip or 10 µg/20 µg soluble recombinant wild type mouse IL-2 (PeproTech, Rocky Hill, NJ), equivalent to the amount of IL-2 coupled on IL-2-Fc-Lip). On day 12 mice were sacrificed and T-cells from inguinal lymph nodes were collected and surface stained for CD8 and vβ13 before analyzing by flow cytometry on a BD FACS Canto to assess the percentage of tumor-specific T-cells in each group.

***Sample preparation for flow cytometry:*** Inguinal lymph nodes and spleens were ground through a 70 µm cell strainer and washed once with ice cold PBS. Splenocytes were then lysed with ACK lysis buffer (2 ml per spleen) for 5 min at 25°C to remove red blood cells before washing in ice cold PBS. Blood samples were lysed with 2X 1ml ACK lysis buffer for 5 min at 25°C and then washed 1x with ice cold PBS. All cells were washed in FACS buffer (PBS with 2% Fetal Calf Serum) once before surface-staining with Ab. After staining, cells were washed 2x in FACS buffer and analyzed on a BD FACS Canto Flow Cytometer. All data was processed using FlowJo software.

***Statistical analysis:*** Statistical analysis was done using GraphPad Prism software and two-tailed unpaired t-tests were conducted between groups of experimental data. Graphs show the mean ± SEM of sample groups.

### Results and Discussion

***Synthesis and Characterization of IL-2-Fc-Lip and anti-Thy1.1-Lip:*** To generate cytokine- or antibody (Ab)-conjugated liposomes (IL-2-Fc-Lip or anti-Thy1.1-Lip) for T-cell targeting, PEGylated liposomes incorporating maleimide-headgroup PEG-lipids (Mal-PEG-DSPE) were prepared from high-Tm lipids and sized by membrane filtration to a mean diameter of 173±13 nm (FIG. 1A, B). Murine IL-2 fused to the C-terminus of mouse IgG2a Fc or anti-Thy1.1 F(ab')2 were coupled to the maleimide termini of PEG chains to serve as targeting ligands of the immunoliposomes. To minimize interaction of liposomes with phagocyte Fc receptors, a D265A mutation was introduced in the Fc portion of IL-2-Fc [24] and F(ab')2 fragments of anti-Thy1.1 monoclonal antibodies were generated by pepsin digestion. Prior to F(ab')2/cytokine coupling, IL-2-Fc and anti-Thy1.1 F(ab')2 were mildly reduced by DTT to expose hinge region free thiols for reaction with the liposome maleimide functional head groups. We tested liposomes containing two different mole fractions of maleimide-PEG lipid (1 or 2.5 mol% of total lipids), and found greater targeting ligand conjugation with higher fractions of maleimide-lipid, as expected (FIG. 1C). Negligible IL-2 or F(ab')2 binding to liposomes was observed in the absence of the maleimide reactive groups. Liposomes with 2.5 mol% mal-PEG-DSPE gave 23 ± 2 µg of IL-2 (cytokine equivalent, or 1.4 nmol IL-2) or 76 ± 7 µg of anti-Thy1.1 (0.5 nmol F(ab')2) per mg lipid after overnight coupling at 25°C. As shown in FIG. 1B, targeting ligand conjugation caused a slight increase in the mean size of the vesicles from 173 ± 13 nm to 186 ± 16 nm.

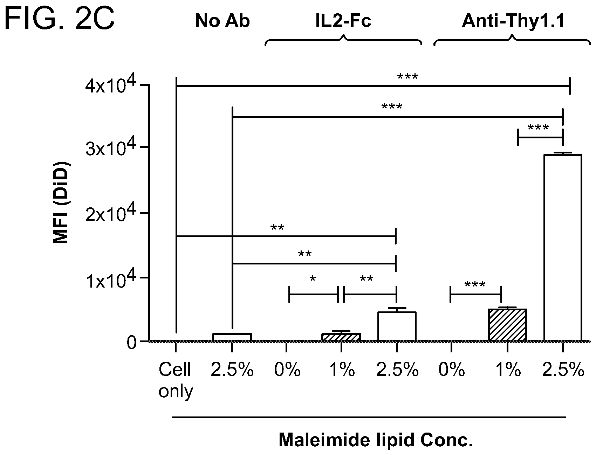

***IL-2-Fc-Lip and anti-Thy1.1-Lip binding to T-cells in vitro:*** To generate a target population of T-cells to be used in adoptive transfer studies, CD8+ T-cells from pmel-1 Thy1.1+ mice (which express a transgenic T-cell receptor specific for the gp100 antigen of melanoma [25]) were isolated by magnetic negative selection from activated splenocytes, and expanded by culturing with IL-2 for 1 day to obtain an elevated expression of CD25 (the α-chain of the trimeric IL-2 receptor) compared to naive pmel-1 or naive polyclonal C57B1/6 CD8+ T-cells (FIG. 2A and data not shown). Fluorescently labeled PEGylated vesicles showed very low background binding to activated pmel-1 T-cells following a 30 min incubation at 37° *in vitro,* but IL-2-Fc-Lip or anti-Thy1.1-Lip containing 1% or 2.5% maleimide functional groups efficiently bound to activated pmel-1 T-cells (FIG. 2B). The Mean Fluorescence Intensities (MFI) of cells after conjugation with different types of liposomes was quantified; the high expression levels of Thy1.1 on pmel-1 T-cells led to much greater per-cell binding of anti-Thy1.1-Lip vs. IL-2-Fc-Lip (FIG. 2C). For both targeting ligands, liposomes containing 2.5 mol% mal-PEG-DSPE (and therefore with higher ligand densities) achieved much greater binding to T-cells than vesicles with 1 mol% of the maleimide lipid, with MFIs of bound liposomes increased by 6-fold and 4-fold for anti-Thy1.1-Lip and IL-2-Fc-Lip, respectively (FIG. 2B, C).

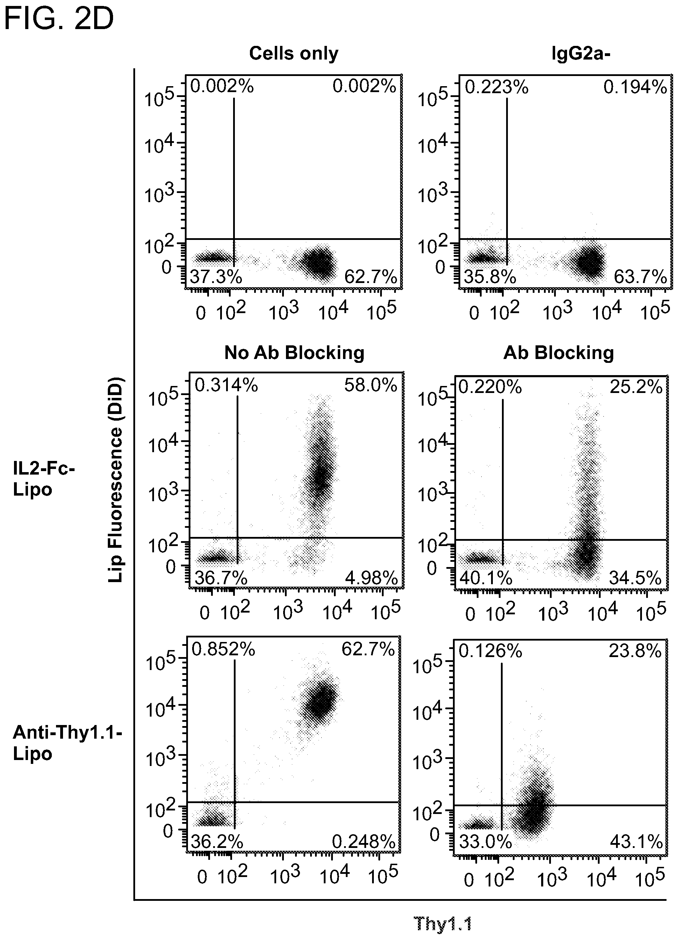

To evaluate the specificity of anti-Thy1.1-Lip and IL-2-Fc-Lip binding, we assessed T-cell labeling in the presence of competing free IL-2-Fc or anti-Thy1.1 Abs added to a 1:1 mixture of naive C57B1/6 lymphocytes and pmel-1 T-cells 30 min before the targeted vesicles. IL-2-Fc-Lip bound to activated pmel-1 T-cells, but not naive C57B1/6 CD8+ T cells that lack IL-2 receptors (FIG. 2D middle left). Pre-blocking pmel-1 T-cells with soluble IL-2-Fc blocked 90% of binding to pmel-1 T-cells (FIG. 2D middle right, 2E). Similarly, anti-Thy1.1-Lip selectively targeted pmel-1 CD8+ T cells but not naive C57B1/6 (Thy1.2+) CD8+ T cells (FIG. 2D bottom left). Pre-incubation of pmel-1 T-cells with anti-Thy1.1 lowered anti-Thy1.1-Lip binding by 99% (FIG. 2D bottom right, 2E). Autofluorescence and non-specific binding of non-targeted control IgG2a-Lip were neglibible (FIG.2D top left and right, 2E). As expected from the pM affinity of IL-2 for its receptor [26, 27] and the typical nM affinity of commercial antibodies, liposomes at concentration of 0.4 mg/ml (equivalent to 2 nM of liposomes) labeled 100% of activated pmel-1 T-cells *in vitro,* and liposome binding reached a plateau at concentrations higher than 0.4 mg/ml (equivalent to 2 nM liposomes) (FIG. 2F). Thus, both IL-2- and anti-Thy1.1-targeted stealth liposomes achieve specific and avid binding to primed pmel-1 CD8+ T-cells. Even when the concentration was titrated down to 0.1mg/ml, nearly 100% of cells were still labeled with liposomes, albeit with fewer liposomes bound per cell.

***Internalization of Anti-Thy1.1-conjugated liposomes:*** We previously reported that IL-2-Fc-conjugated liposomes are rapidly internalized by activated T-cells *in vitro* [28]. To determine whether anti-Thy1.1-Lip would also trigger liposome endocytosis, we added anti-Thy1.1-Lip incorporating a carboxyfluorescein (CF)-headgroup lipid to pmel-1 T-cells at 4°C to allow binding without internalization, then warmed the cells to 37°C and assessed cell-associated fluorescence over time. Fluorescein has highly pH-sensitive fluorescence that is strongly quenched at acidic pHs [29]. The high avidity of liposome binding to cells led to no measurable release of free liposomes into the supernatant over 6 hr at 37°C (not shown); we thus attributed loss of the CF tracer signal to endocytic uptake by labeled cells. Over a time course of 6 hr, the MFI of liposome-labeled T-cells steadily dropped, corresponding to roughly 90% internalization over this time course (Fig 3A). Confocal imaging also showed that anti-Thy1.1-Lip fluorescence initially localized to the plasma membrane of labeled cells was largely lost by 6 hr (FIG. 3B).

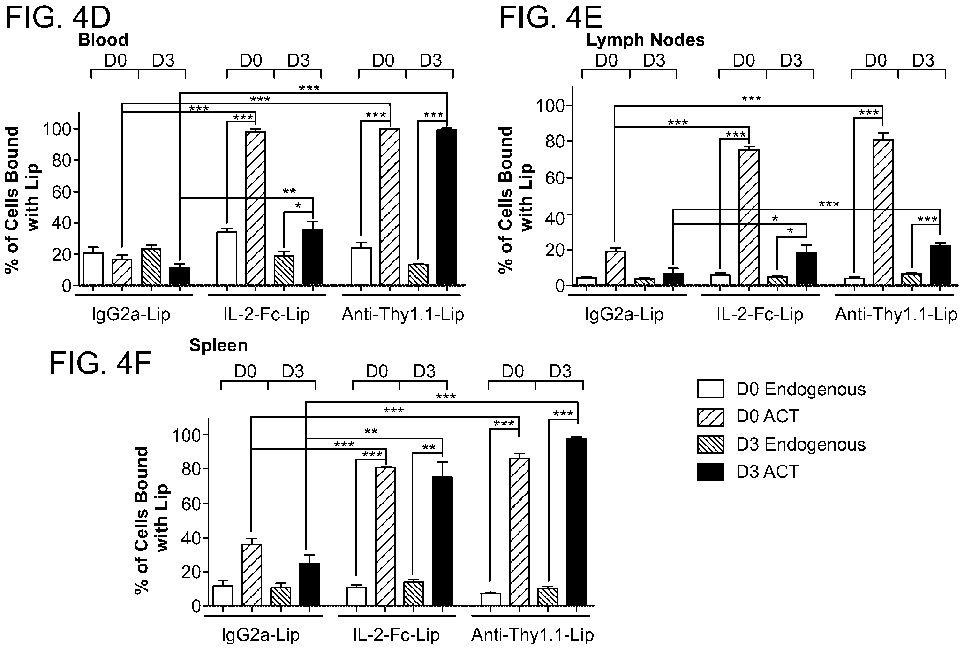

***In vivo targeting of IL-2-Fc-Lip and anti-Thy1.1-Lip in healthy animals:*** Next, we tested the capacity of anti-Thy1.1-Lip and IL-2-Fc-Lip to target pmel-1 T-cells *in vivo* in healthy mice. PEGylated liposomes conjugated with isotype control murine IgG2a were prepared to serve as a control non-T-cell-targeting liposome. To model clinical adoptive T-cell therapy, recipient Thy1.2+ C57B1/6 mice were lymphodepleted by sublethal irradiation, followed by i.v. injection of 15x10⁶ activated pmel-1 Thy1.1+CD8+ T-cells one day later. Lymphodepletion removes cytokine sinks and regulatory T-cells to allow more efficient expansion and effector function of transferred T-cells [30, 31]. To assess T-cell targeting, IgG2a-Lip, IL-2-Fc-Lip, or anti-Thy1.1-Lip fluorescently labeled with the non-pH-sensitive tracer DiD were injected i.v. either immediately after adoptive transfer or 3 days after T-cell injection. Twenty-four hours after liposome injection, cells from the blood, lymph nodes (LNs), and spleens were analyzed by flow cytometry to assess binding of fluorescent liposomes (FIG. 4A). Thy1.1 expression allowed liposome binding to transferred pmel-1 T-cells to be distinguished from endogenous T-cells (FIG. 4B). Sample flow cytometry histograms are shown in FIG. 4C, illustrating conjugation efficiencies of IgG2a-Lip, IL-2-Fc-Lip, and anti-Thy1.1-Lip obtained when liposomes were injected immediately after ACT T-cells. The percentage endogenous or ACT CD8+ T-cells labeled by each type of liposome in the blood (FIG. 4D), lymph nodes (FIG. 4E) and spleens (FIG. 4F) were assessed; this analysis revealed that IgG2a-Lip exhibited low binding to both T-cell populations. In contrast, anti-Thy1.1-Lip labeled nearly 100% of the transferred T-cells in the blood and spleen whether injected on day 0 or day 3. The slightly greater background binding of isotype control IgG2a-Lip to ACT vs. endogenous T-cells in spleens was found to be an artifact of the liposome dose; injection of lower liposome doses of 0.18 mg (approximately ∼0.1 mg/mL liposomes in the blood) led to a similar efficiency of specific T-cell binding but eliminated the low differential background binding to ACT vs. endogenous T-cells (data not shown). A lower fraction of T-cells in lymph nodes were labeled by anti-Thyl. 1-Lip following a day 3 injection, which may reflect a combination of poor entry of targeted liposomes into LN and/or incomplete recirculation of T-cells from LN back into the blood in the 24 hr time window between liposome injection and our analysis. Anti-Thy1.1-Lip also showed low levels of background binding to endogenous (Thy1.1-) T-cells. IL-2-Fc-Lip labeled the majority of pmel-1 T-cells in the LNs, spleen and blood when injected just after T-cells, and also showed relatively low binding to endogenous T-cells. However, injection of IL-2Fc-Lip on day 3 led to relatively poor T-cell labeling in the blood and LNs, while still labeling a majority of ACT T-cells in the spleen. Poor labeling by IL-2-Fc-Lip on day 3 reflected rapid downregulation of the IL-2R *in vivo* following T-cell transfer in the absence of antigen (data not shown). Thus, both IL-2-Fc and anti-Thy1.1 F(ab')2 can be effective for specifically targeting adoptively transferred T-cells *in vivo.*

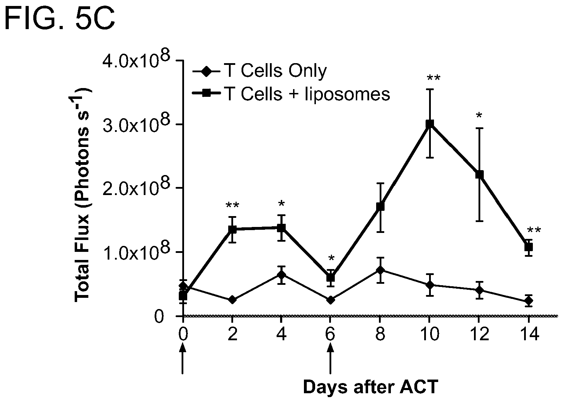

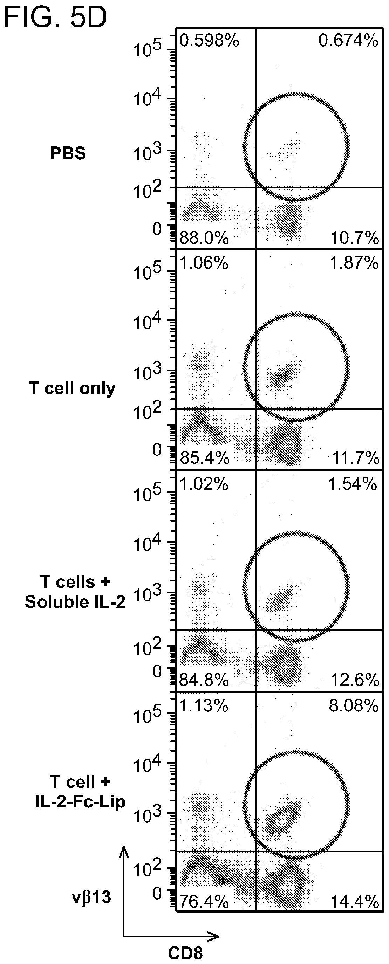

***IL-2-Fc-Lip permit repeated boosting of ACT T-cells in a murine lung metastasis model:*** To test the potential functional impact of stimulatory T-cell targeted liposomes, we assessed the response of pmel-1 melanoma-specific T-cells *in vivo* during ACT treatment of B16F10 tumors in a metastatic lung tumor model. B16F10 melanoma cells were injected via the tail vein to allow lung metastases to establish for 10 days, then animals were lymphodepleted and received adoptive transfer of luciferase-expressing pmel-1 melanoma-specific CD8+ T-cells (FIG. 5A). In one group of animals, T-cell expansion was followed over time by bioluminescence imaging without further treatment, while in other two groups of mice, the adoptively-transferred cells were boosted on days 0 and 6 by injection of IL-2-Fc-Lip. Adoptively transferred cells without further adjuvant support showed a low level persistence in the tumor-bearing recipients that gradually declined over 14 days, as expected in the absence of additional stimulation or protection from tumor immunosuppression [25] (Fig 5B, C). In contrast, following injection of the first dose of IL-2-Fc-Lip, pmel-1 T-cells expanded 3-fold more than the control T-cell therapy group. These boosted T-cells began to contract again between day 4 and day 6, but following a second dose of IL-2-Fc-Lip, re-expanded to an even greater level, reaching a peak by day 10 with 6-fold greater T-cell numbers relative to the T-cell-only treatment group (Fig 5B, C). To assess the relative potency of stimulation achieved by IL-2-Fc-Lip compared to traditional systemic IL-2 therapy, we repeated this ACT experiment and compared the expansion of T-cells following injection of IL-2-Fc-Lip or soluble IL-2 (at an equivalent total amount of cytokine to that bound to the liposomes) on day 0 and day 6. Flow cytometry analysis of T-cells pooled from the inguinal lymph nodes 12 days after adoptive transfer confirmed that the frequency of tumor-specific CD8+ T-cells (pmel-1 T-cells express the Vβ13 TCR β chain) was nearly 3 times greater in animals that received IL-2-Fc-Lip injections compared to T-cells alone (Figs. 5D-E). Further, soluble IL-2 at these doses showed no enhancement in T-cell expansion. The difference between the potency of IL-2-Fc-Lip and soluble IL-2 may reflect the very short half-life of IL-2 *in vivo* [32], which the PEGylated liposomes may partly overcome. Notably, this enhanced potency was not accompanied by overt toxicity as assessed by changes in animal weights during the therapy (data not shown). Thus, IL-2-targeted liposomes allow multiple boosts of ACT T-cells *in vivo,* leading to repeated waves of T-cells expansion in tumor-bearing animals, which exceed the response elicited by systemic free IL-2.

Here we synthesized and characterized antibody- and cytokine-decorated immunoliposomes targeting unique cell surface antigens or activation markers on T-cells, respectively. Targeting liposomes bound to ACT T-cells specifically *in vitro,* and further, anti-Thy1.1-Lip also labeled nearly 100% of transferred T-cells in systemic compartments and most of transferred T-cells in LN *in vivo* following a single injection of targeted vesicles. Despite its lower targeting specificity compared to anti-Thy1.1-Lip, IL-2-Fc-Lip was able to repeatedly boost transferred T-cells *in vivo* in tumor-bearing animals and provide direct stimulation to ACT T-cells. These results demonstrate the concept of repeated targeting of ACT T-cells for adjuvant stimulation *in vivo.* Also envisioned is functional targeting of supporting adjuvant drugs or imaging contrast agents to T-cells, in order to enhance the efficacy of ACT and/or permit longitudinal tracking of ACT T-cells *in vivo.*

### Example 2

This Example provides data obtained from systemic delivery of anti-CD 137-conjugated liposomes and IL-2-Fc-conjugated liposomes.

Antibody-conjugated liposomes were spherical and formed by single lipid bilayer, with the particle sizes of 30-50 nm (FIG. 6B), and their zeta potentials were around -30 mV.

In B16-OVA subcutaneous tumor model, 5×10⁵ B16-OVA cells were inoculated to the flank of the mice. When the tumors reached -100 mm³, mice were given intravenous (i.v.) injections of soluble CD137/IL-2-Fc or Lipo-CD137/Lipo-IL-2-Fc on day 0, 2 and 4 with a 100 µg/dose of αCD137 and a 20 µg/dose of IL-2-Fc. Isotype control IgG conjugated liposome (Lipo-IgG) was used as the control liposome. Both soluble CD137/IL-2-Fc and Lipo-CD137/Lipo-IL-2-Fc significantly suppressed the tumor growth (FIG. 7A). The mice in the soluble CD137/IL-2-Fc group started to lose body weight right after the treatment, and half of them died on day 11. By contrast, all the mice in Lipo-CD137/Lipo-IL-2-Fc remained in good physical condition during the treatment, and they all survived after two weeks with little rebound in tumor burden (FIGs. 7B and 7C).

On day 6 post injection, CD8⁺ T cells were analyzed from lymphocytes in the peripheral blood. Soluble CD137/IL-2-Fc treatment induced 10 folds CD8⁺ T cell enrichment in PBMC comparing to the untreated group, while Lipo-CD137/ Lipo-IL-2-Fc triggered 5 folds CD8⁺ T cell enrichment. Lipo-IgG had no effect on CD8⁺ T cell number in PBMC (FIG. 8).

On day 6 post injection, lymphocytes from PBMC were pulsed with 10 µM OVA protein for 8 hours, followed by addition of brefeldin A for 5 hours. Then, the intracellular staining of IPNγ and TNFα was analyzed by flow cytometry. Soluble CD137/IL-2-Fc dramatically triggered IFNγ and TNFα production, an about 30-fold increase relative to untreated group, in terms of total number of IFNγ⁺/TNFα⁺ CD8+⁺ cells. Lipo-CD137/ Lipo-IL-2-Fc also induced an about 10-fold increase in IPNγ and TNFα production in CD8⁺ T cells relative to the untreated group. Lipo-IgG had little effect on the intracellular cytokine production (FIGs. 9A-9B).

In a B16F10 subcutaneous tumor model, 5×10⁵ B16F10 cells were inoculated to the flank of the mice. When the tumors reached ∼60 mm³, mice were given i.v. injections of soluble CD137/IL-2-Fc or Lipo-CD137/Lipo-IL-2-Fc on day 0, 3 and 6 with a 100 µg/dose of αCD137 and a 60 µg/dose of IL-2-Fc. Isotype control IgG conjugated liposome (Lipo-IgG) was used as the control liposome. Lipo-CD137/Lipo-IL-2-Fc significantly retarded the tumor growth comparing to untreated and Lipo-IgG groups. Soluble CD137/IL-2-Fc controlled the tumor growth in the early stage, but after the second i.v. injection, all the mice in soluble CD137/IL-2-Fc group died (FIG. 10C) due to the severe *in vivo* toxicity which coincided with their dramatic body weight loss right after the treatment (FIG. 10B). Unexpectedly, all the mice in Lipo-CD137/Lipo-IL-2-Fc remained good physical condition during the treatment, and all the mice survived during the therapeutic study (FIGs. 10B-10C).

Two days after a single i.v. injection in B16F10 tumor bearing mice, blood serums were collected, and serum cytokine levels were measured by LUMINEX® cytokine bead assay. Soluble CD137/IL-2-Fc triggered a dramatic increase in inflammatory cytokine levels, including IPNγ, IL6, MCP-1 and TNFα, which led to lethal *in vivo* toxicities observed in the therapeutic studies. Lipo-CD137/Lipo-IL-2-Fc had little effect on the elevation of inflammatory cytokine levels, indicating systemic delivery of Lipo-CD137/Lipo-IL-2-Fc prevented lethal systemic inflammatory toxicity (FIG. 11).

### REFERENCES

[1] I. Mellman, G. Coukos, G. Dranoff, Cancer immunotherapy comes of age, Nature, 480 (2011) 480-489.
[2] S.L. Topalian, F.S. Hodi, J.R. Brahmer, S.N. Gettinger, D.C. Smith, D.F. McDermott, J.D. Powderly, R.D. Carvajal, J.A. Sosman, M.B. Atkins, P.D. Leming, D.R. Spigel, S.J. Antonia, L. Horn, C.G. Drake, D.M. Pardoll, L. Chen, W.H. Sharfman, R.A. Anders, J.M. Taube, T.L. McMiller, H. Xu, A.J. Korman, M. Jure-Kunkel, S. Agrawal, D. McDonald, G.D. Kollia, A. Gupta, J.M. Wigginton, M. Sznol, Safety, activity, and immune correlates of anti-PD-1 antibody in cancer, The New England journal of medicine, 366 (2012) 2443-2454.
[3] M. Kalos, B.L. Levine, D.L. Porter, S. Katz, S.A. Grupp, A. Bagg, C.H. June, T cells with chimeric antigen receptors have potent antitumor effects and can establish memory in patients with advanced leukemia, Science translational medicine, 3 (2011) 95ra73.
[4] S.A. Rosenberg, J.C. Yang, R.M. Sherry, U.S. Kammula, M.S. Hughes, G.Q. Phan, D.E. Citrin, N.P. Restifo, P.F. Robbins, J.R. Wunderlich, K.E. Morton, C.M. Laurencot, S.M. Steinberg, D.E. White, M.E. Dudley, Durable complete responses in heavily pretreated patients with metastatic melanoma using T-cell transfer immunotherapy, Clinical cancer research : an official journal of the American Association for Cancer Research, 17 (2011) 4550-4557.
[5] C. Yee, J.A. Thompson, D. Byrd, S.R. Riddell, P. Roche, E. Celis, P.D. Greenberg, Adoptive T cell therapy using antigen-specific CD8+ T cell clones for the treatment of patients with metastatic melanoma: in vivo persistence, migration, and antitumor effect of transferred T cells, Proceedings of the National Academy of Sciences of the United States of America, 99 (2002) 16168-16173.
[6] R.A. Morgan, M.E. Dudley, J.R. Wunderlich, M.S. Hughes, J.C. Yang, R.M. Sherry, R.E. Royal, S.L. Topalian, U.S. Kammula, N.P. Restifo, Z. Zheng, A. Nahvi, C.R. de Vries, L.J. Rogers-Freezer, S.A. Mavroukakis, S.A. Rosenberg, Cancer regression in patients after transfer of genetically engineered lymphocytes, Science, 314 (2006) 126-129.
[7] N.P. Restifo, M.E. Dudley, S.A. Rosenberg, Adoptive immunotherapy for cancer: harnessing the T cell response, Nature reviews. Immunology, 12 (2012) 269-281.
[8] M.J. Besser, R. Shapira-Frommer, A.J. Treves, D. Zippel, O. Itzhaki, L. Hershkovitz, D. Levy, A. Kubi, E. Hovav, N. Chermoshniuk, B. Shalmon, I. Hardan, R. Catane, G. Markel, S. Apter, A. Ben-Nun, I. Kuchuk, A. Shimoni, A. Nagler, J. Schachter, Clinical responses in a phase II study using adoptive transfer of short-term cultured tumor infiltration lymphocytes in metastatic melanoma patients, Clinical cancer research : an official journal of the American Association for Cancer Research, 16 (2010) 2646-2655.
[9] M.P. Rubinstein, C.A. Cloud, T.E. Garrett, C.J. Moore, K.M. Schwartz, C.B. Johnson, D.H. Craig, M.L. Salem, C.M. Paulos, D.J. Cole, Ex vivo interleukin-12-priming during CD8(+) T cell activation dramatically improves adoptive T cell transfer antitumor efficacy in a lymphodepleted host, Journal of the American College of Surgeons, 214 (2012) 700-707; discussion 707-708.
[10] P.A. Prieto, K.H. Durflinger, J.R. Wunderlich, S.A. Rosenberg, M.E. Dudley, Enrichment of CD8+ cells from melanoma tumor-infiltrating lymphocyte cultures reveals tumor reactivity for use in adoptive cell therapy, J Immunother, 33 (2010) 547-556.
[11] H. Kobayashi, Y. Tanaka, J. Yagi, N. Minato, K. Tanabe, Phase I/II study of adoptive transfer of gammadelta T cells in combination with zoledronic acid and IL-2 to patients with advanced renal cell carcinoma, Cancer immunology, immunotherapy : CII, 60 (2011) 1075-1084.
[12] J.C. Markley, M. Sadelain, IL-7 and IL-21 are superior to IL-2 and IL-15 in promoting human T cell-mediated rejection of systemic lymphoma in immunodeficient mice, Blood, 115 (2010) 3508-3519.
[13] S.P. Kerkar, P. Muranski, A. Kaiser, A. Boni, L. Sanchez-Perez, Z. Yu, D.C. Palmer, R.N. Reger, Z.A. Borman, L. Zhang, R.A. Morgan, L. Gattinoni, S.A. Rosenberg, G. Trinchieri, N.P. Restifo, Tumor-specific CD8+ T cells expressing interleukin-12 eradicate established cancers in lymphodepleted hosts, Cancer research, 70 (2010) 6725-6734.
[14] C. Hsu, S.A. Jones, C.J. Cohen, Z. Zheng, K. Kerstann, J. Zhou, P.F. Robbins, P.D. Peng, X. Shen, T.J. Gomes, C.E. Dunbar, D.J. Munroe, C. Stewart, K. Cornetta, D. Wangsa, T. Ried, S.A. Rosenberg, R.A. Morgan, Cytokine-independent growth and clonal expansion of a primary human CD8+ T-cell clone following retroviral transduction with the IL-15 gene, Blood, 109 (2007) 5168-5177.
[15] M.T. Stephan, J.J. Moon, S.H. Um, A. Bershteyn, D.J. Irvine, Therapeutic cell engineering with surface-conjugated synthetic nanoparticles, Nature medicine, 16 (2010) 1035-1041.
[16] M.T. Stephan, S.B. Stephan, P. Bak, J. Chen, D.J. Irvine, Synapse-directed delivery of immunomodulators using T-cell-conjugated nanoparticles, Biomaterials, 33 (2012) 5776-5787.
[17] T.M. Fahmy, J.P. Schneck, W.M. Saltzman, A nanoscopic multivalent antigen-presenting carrier for sensitive detection and drug delivery to T cells, Nanomedicine, 3 (2007) 75-85.
[18] S. Tsai, A. Shameli, J. Yamanouchi, X. Clemente-Casares, J. Wang, P. Serra, Y. Yang, Z. Medarova, A. Moore, P. Santamaria, Reversal of autoimmunity by boosting memory-like autoregulatory T cells., Immunity, 32 (2010) 568-580.
[19] X. Clemente-Casares, S. Tsai, Y. Yang, P. Santamaria, Peptide-MHC-based nanovaccines for the treatment of autoimmunity: a "one size fits all" approach?, Journal of molecular medicine (Berlin, Germany), 89 (2011) 733-742.
[20] X. Wang, W.C. Chang, C.W. Wong, D. Colcher, M. Sherman, J.R. Ostberg, S.J. Forman, S.R. Riddell, M.C. Jensen, A transgene-encoded cell surface polypeptide for selection, in vivo tracking, and ablation of engineered cells, Blood, 118 (2011) 1255-1263.
[21] M. Kalos, Biomarkers in T cell therapy clinical trials, Journal of translational medicine, 9 (2011) 138.
[22] Y. Minami, T. Kono, T. Miyazaki, T. Taniguchi, The IL-2 receptor complex: its structure, function, and target genes, Annual review of immunology, 11 (1993) 245-268.
[23] B. Kwong, S.A. Gai, J. Elkhader, K.D. Wittrup, D.J. Irvine, Localized immunotherapy via liposome-anchored Anti-CD 137 + IL-2 prevents lethal toxicity and elicits local and systemic antitumor immunity, Cancer research, 73 (2013) 1547-1558.
[24] L. Baudino, Y. Shinohara, F. Nimmerjahn, J. Furukawa, M. Nakata, E. Martinez-Soria, F. Petry, J.V. Ravetch, S. Nishimura, S. Izui, Crucial role of aspartic acid at position 265 in the CH2 domain for murine IgG2a and IgG2b Fc-associated effector functions, Journal of immunology, 181 (2008) 6664-6669.
[25] W.W. Overwijk, M.R. Theoret, S.E. Finkelstein, D.R. Surman, L.A. de Jong, F.A. Vyth-Dreese, T.A. Dellemijn, P.A. Antony, P.J. Spiess, D.C. Palmer, D.M. Heimann, C.A. Klebanoff, Z. Yu, L.N. Hwang, L. Feigenbaum, A.M. Kruisbeek, S.A. Rosenberg, N.P. Restifo, Tumor regression and autoimmunity after reversal of a functionally tolerant state of self-reactive CD8+ T cells, The Journal of experimental medicine, 198 (2003) 569-580.
[26] J.W. Lowenthal, P. Corthesy, C. Tougne, R. Lees, H.R. MacDonald, M. Nabholz, High and low affinity IL 2 receptors: analysis by IL 2 dissociation rate and reactivity with monoclonal anti-receptor antibody PC61, Journal of immunology, 135 (1985) 3988-3994.
[27] J.W. Lowenthal, R.H. Zubler, M. Nabholz, H.R. MacDonald, Similarities between interleukin-2 receptor number and affinity on activated B and T lymphocytes, Nature, 315 (1985) 669-672.
[28] B. Kwong, Liposome-anchored local delivery of immunomodulatory agents for tumor therapy, in: Biological Engineering, Massachusetts Institute of Technology, http://hdl.handle.net/1721.1/76115, 2012.
[29] R.F. Murphy, S. Powers, C.R. Cantor, Endosome pH measured in single cells by dual fluorescence flow cytometry: rapid acidification of insulin to pH 6, The Journal of cell biology, 98 (1984) 1757-1762.
[30] C.A. Klebanoff, H.T. Khong, P.A. Antony, D.C. Palmer, N.P. Restifo, Sinks, suppressors and antigen presenters: how lymphodepletion enhances T cell-mediated tumor immunotherapy, Trends in immunology, 26 (2005) 111-117.
[31] M.E. Dudley, J.R. Wunderlich, J.C. Yang, P. Hwu, D.J. Schwartzentruber, S.L. Topalian, R.M. Sherry, F.M. Marincola, S.F. Leitman, C.A. Seipp, L. Rogers-Freezer, K.E. Morton, A. Nahvi, S.A. Mavroukakis, D.E. White, S.A. Rosenberg, A phase I study of nonmyeloablative chemotherapy and adoptive transfer of autologous tumor antigen-specific T lymphocytes in patients with metastatic melanoma, J Immunother, 25 (2002) 243-251.
[32] M.W. Konrad, G. Hemstreet, E.M. Hersh, P.W. Mansell, R. Mertelsmann, J.E. Kolitz, E.C. Bradley, Pharmacokinetics of recombinant interleukin 2 in humans, Cancer research, 50 (1990) 2009-2017.
[33] Hodi, F. S. et al. Improved survival with ipilimumab in patients with metastatic melanoma. N Engl J Med 363, 711-723 (2010).
[34] Topalian, S. L. et al. Safety, Activity, and Immune Correlates of Anti-PD-1 Antibody in Cancer. N Engl J Med 366, 2443-2454 (2012).
[35] ZHENG, Y ET AL.: "In Vivo Targeting Of Adoptively Transferred T-Cells With Antibody- And Cytokine-Conjugated Liposomes.", J. CONTROL RELEASE., vol. 172, no. 2, 11 June 2013 (2013-06-11), pages 426-435, DOI: 10.1016/ J.JCONREL.2013.05.037.

## Claims

1. A population of lymphocyte-targeting liposomes for use in a therapeutic method in a subject, wherein the population comprises (a) lymphocyte-targeting liposomes comprising a lymphocyte specific ligand, wherein the lymphocyte specific ligand is a cytokine that stimulates lymphocytes; and (b) lymphocyte-targeting liposomes comprising an antibody or antibody fragment that binds to CD137, CTLA-4 or PD-1; and wherein said population of lymphocyte-targeting liposomes is administered to the subject in need thereof by repeated systemic administration.

2. A population of lymphocyte-targeting liposomes for use in a therapeutic method in a subject, wherein the individual lymphocyte-targeting liposomes comprise on their surface (i) a lymphocyte specific ligand, wherein the lymphocyte specific ligand is a cytokine that stimulates lymphocytes; and (ii) an antibody or antibody fragment that binds to CD137, CTLA-4 or PD-1; and wherein said population of lymphocyte-targeting liposomes is administered to the subject in need thereof by repeated systemic administration.

3. The population of lymphocyte-targeting liposomes for use according to any one of claims 1-2, wherein the lymphocyte-targeting liposomes further comprise an active agent; e.g. wherein the active agent is encapsulated in the lymphocyte-targeting liposomes; or wherein the active agent is bound to a surface of the lymphocyte-targeting liposomes.

4. The population of lymphocyte-targeting liposomes for use according to any one of claims 1-3, wherein the lymphocyte-targeting liposomes target endogenous T cells, e.g. wherein the lymphocyte-targeting liposomes stimulate activity and/or proliferation of endogenous T cells; and/or wherein the lymphocyte-targeting liposomes target adoptively-transferred T-cells or T cells engineered to express a T cell receptor.

5. The population of lymphocyte-targeting liposomes for use according to any one of claims 1-4, wherein the cytokine is IL-2, IL-7, IL-15, or superagonist/mutant forms thereof, or an Fc-fusion protein of any one of the foregoing cytokines.

6. The population of lymphocyte-targeting liposomes for use according to any one of claims 3-5, wherein the active agent is a chemical entity, a protein, a polypeptide, a peptide, a nucleic acid, a virus-like particle, a steroid, a proteoglycan, a lipid or a carbohydrate; and/or wherein the active agent is a therapeutic agent; and/or wherein the active agent is an agent that inhibits immunosuppression, e.g. wherein the active agent that inhibits immunosuppression is a Shp1/2 protein tyrosine phosphatase (PTPase) inhibitor.

7. The population of lymphocyte-targeting liposomes for use according to any one of claims 1-6, wherein the lymphocyte-targeting liposomes are PEGylated lymphocyte-targeting liposomes; and/or wherein the lymphocyte-targeting particles are polymer-based lymphocyte-targeting particles.

8. The population of lymphocyte-targeting liposomes for use according to any one of claims 1-7, wherein repeated administration comprises daily, weekly or biweekly administration.

9. The population of lymphocyte-targeting liposomes for use according to any one of claims 1-8, wherein the subject has cancer; and/or wherein the subject has an infection.

10. The population of lymphocyte-targeting liposomes for use according to of any one of claims 1-9, wherein the subject is undergoing or has undergone adoptive cell therapy.

11. The population of lymphocyte-targeting liposomes for use according to of any one of claims 1-10, wherein repeated administration comprises administration substantially simultaneously with the administration of tumor-reactive T cells, and at least one administration after administration of tumor-reactive T cells.

12. The population of lymphocyte-targeting liposomes for use according to of any one of claims 1-11, wherein the subject is undergoing or has undergone adoptive cell therapy and wherein the adoptive cell therapy comprises administration of CD8+ T cells.

13. The population of lymphocyte-targeting liposomes for use according to any one of claims 1-12, wherein the liposomes are administered parenterally; e.g. wherein the liposomes are administered intravenously or subcutaneously; such as wherein the liposomes are formulated for injection in unit dosage form or multi-dose containers.

14. The population of lymphocyte-targeting liposomes for use according to any one of claims 1-13, wherein the liposomes are administered at least twice, at least 3 times, at least 4 times, at least 5 times, at least 6 times, at least 7 times, at least 8 times, or more; and/or wherein the liposomes are administered regularly or randomly spaced in time; and/or wherein the liposomes are administered days apart, or weeks apart or months apart; and/or wherein the liposomes are administered every day, every 2 days, every 3 days, every 4 days, every 5 days, every 6 days, every week, every 2 weeks, every 3 weeks, or every 4 weeks.

15. The population of lymphocyte-targeting liposomes for use according to any one of claims 1-14, wherein the liposomes are formulated in a pharmaceutical composition in the form of a plurality of doses.

## Patentansprüche

1. Eine Population von auf Lymphozyten ausgerichteten Liposomen für eine Verwendung im Rahmen eines therapeutischen Verfahrens in einem Subjekt, die Population umfassend (a) auf Lymphozyten ausgerichtete Liposome, die einen lymphozytenspezifischen Liganden umfassen, wobei der lymphozytenspezifische Ligand ein Zytokin ist, das Lymphozyten stimuliert; und (b) auf Lymphozyten ausgerichtete Liposome, die einen Antikörper oder ein Antikörperfragment umfassen, der/das an CD137, CTLA-4 oder PD-1 bindet; und wobei die Population von auf Lymphozyten ausgerichteten Liposomen dem Subjekt, das einer solchen Verabreichung bedarf, durch wiederholte systemische Verabreichung verabreicht wird.

2. Eine Population von auf Lymphozyten ausgerichteten Liposomen für eine Verwendung im Rahmen eines therapeutischen Verfahrens in einem Subjekt, wobei die einzelnen auf Lymphozyten ausgerichteten Liposome auf ihrer Oberfläche (i) einen lymphozytenspezifischen Liganden umfassen, wobei der lymphozytenspezifische Ligand ein Zytokin ist, das Lymphozyten stimuliert; und (ii) einen Antikörper oder ein Antikörperfragment, der/das an CD137, CTLA-4 oder PD-1 bindet; und wobei die Population von auf Lymphozyten ausgerichteten Liposomen dem Subjekt, das einer solchen Verabreichung bedarf, durch wiederholte systemische Verabreichung verabreicht wird.

3. Die Population von auf Lymphozyten ausgerichteten Liposomen für eine Verwendung nach einem beliebigen der Ansprüche 1-2, wobei die auf Lymphozyten ausgerichteten Liposome zudem einen Wirkstoff umfassen; z. B., wobei der Wirkstoff in den auf Lymphozyten ausgerichteten Liposomen eingekapselt ist; oder wobei der Wirkstoff an eine Oberfläche der auf Lymphozyten ausgerichteten Liposome gebunden ist.

4. Die Population von auf Lymphozyten ausgerichteten Liposomen für eine Verwendung nach einem beliebigen der Ansprüche 1-3, wobei die auf Lymphozyten ausgerichteten Liposome auf endogene T-Zellen ausgerichtet sind, z. B., wobei die auf Lymphozyten ausgerichteten Liposome die Aktivität und/oder Proliferation von endogenen T-Zellen stimulieren; und/oder wobei die auf Lymphozyten ausgerichteten Liposome auf angenommen übertragene T-Zellen oder T-Zellen ausgerichtet sind, die gentechnisch so verändert wurden, dass sie einen T-Zell-Rezeptor exprimieren.

5. Die Population von auf Lymphozyten ausgerichteten Liposomen für eine Verwendung nach einem beliebigen der Ansprüche 1-4, wobei das Zytokin IL-2, IL-7, IL-15 oder superagonistische/mutierte Formen davon oder ein Fc-Fusionsprotein von einem beliebigen der vorstehend aufgeführten Zytokine ist.

6. Die Population von auf Lymphozyten ausgerichteten Liposomen für eine Verwendung nach einem beliebigen der Ansprüche 3-5, wobei der Wirkstoff eine chemische Einheit, ein Protein, ein Polypeptid, ein Peptid, eine Nukleinsäure, ein virusähnliches Partikel, ein Steroid, ein Proteoglycan, ein Lipid oder ein Kohlenhydrat ist; und/oder wobei der Wirkstoff ein Therapeutikum ist; und/oder wobei der Wirkstoff ein Mittel ist, das die Immunsuppression inhibiert, z. B., wobei der Wirkstoff, der die Immunsuppression inhibiert, ein Shp 12-Protein-Tyrosin-Phosphatase-(PTPase)-Inhibitor ist.

7. Die Population von auf Lymphozyten ausgerichteten Liposomen für eine Verwendung nach einem beliebigen der Ansprüche 1-6, wobei die auf Lymphozyten ausgerichteten Liposome PEGylierte auf Lymphozyten ausgerichtete Liposome sind; und/oder wobei die auf Lymphozyten ausgerichteten Partikel polymerbasierte auf Lymphozyten ausgerichtete Partikel sind.

8. Die Population von auf Lymphozyten ausgerichteten Liposomen für eine Verwendung nach einem beliebigen der Ansprüche 1-7, wobei zu der wiederholten Verabreichung eine tägliche, wöchentliche oder zweiwöchentliche Verabreichung gehört.

9. Die Population von auf Lymphozyten ausgerichteten Liposomen für eine Verwendung nach einem beliebigen der Ansprüche 1-8, wobei das Subjekt an Krebs leidet; und/oder wobei das Subjekt an einer Infektion leidet.

10. Die Population von auf Lymphozyten ausgerichteten Liposomen für eine Verwendung nach einem beliebigen der Ansprüche 1-9, wobei das Subjekt eine Behandlung mit angenommenen Zellen durchläuft oder durchlaufen hat.

11. Die Population von auf Lymphozyten ausgerichteten Liposomen für eine Verwendung nach einem beliebigen der Ansprüche 1-10, wobei zu der wiederholten Verabreichung eine Verabreichung im Wesentlichen zeitgleich mit der Verabreichung von tumorreaktiven T-Zellen und wenigstens eine Verabreichung nach der Verabreichung von tumorreaktiven T-Zellen gehören.

12. Die Population von auf Lymphozyten ausgerichteten Liposomen für eine Verwendung nach einem beliebigen der Ansprüche 1-11, wobei das Subjekt eine Behandlung mit angenommenen Zellen durchläuft oder durchlaufen hat und wobei zu der Behandlung mit angenommenen Zellen die Verabreichung von CD8+-T-Zellen gehört.

13. Die Population von auf Lymphozyten ausgerichteten Liposomen für eine Verwendung nach einem beliebigen der Ansprüche 1-12, wobei die Liposome parenteral verabreicht werden; z. B., wobei die Liposome intravenös oder subkutan verabreicht werden; wie etwa, wobei die Liposome für eine Injektion in Dosiseinheitsform oder mehrere Dosen enthaltenden Behältern formuliert sind.

14. Die Population von auf Lymphozyten ausgerichteten Liposomen für eine Verwendung nach einem beliebigen der Ansprüche 1-13, wobei die Liposome wenigstens zweimal, wenigstens 3-mal, wenigstens 4-mal, wenigstens 5-mal, wenigstens 6-mal, wenigstens 7-mal, wenigstens 8-mal oder mehr verabreicht werden; und/oder wobei die Liposome in regelmäßigen oder zufälligen Zeitabständen verabreicht werden; und/oder wobei die Liposome im Abstand von Tagen oder im Abstand von Wochen oder im Abstand von Monaten verabreicht werden; und/oder wobei die Liposome täglich, alle 2 Tage, alle 3 Tage, alle 4 Tage, alle 5 Tage, alle 6 Tage, wöchentlich, alle 2 Wochen, alle 3 Wochen oder alle 4 Wochen verabreicht werden.

15. Die Population von auf Lymphozyten ausgerichteten Liposomen für eine Verwendung nach einem beliebigen der Ansprüche 1-14, wobei die Liposome in einer pharmazeutischen Zusammensetzung in Form einer Vielzahl von Dosen formuliert sind.

## Revendications

1. Population de liposomes ciblant les lymphocytes destinée à être utilisée dans une méthode thérapeutique chez un sujet, dans laquelle la population comprend (a) des liposomes ciblant les lymphocytes comprenant un ligand spécifique des lymphocytes, dans laquelle le ligand spécifique des lymphocytes est une cytokine qui stimule les lymphocytes ; et (b) des liposomes ciblant les lymphocytes comprenant un anticorps ou un fragment d'anticorps qui se lie à CD137, CTLA-4 ou PD-1 ; et dans laquelle ladite population de liposomes ciblant les lymphocytes est administrée au sujet en ayant besoin par une administration systémique répétée.

2. Population de liposomes ciblant les lymphocytes destinée à être utilisée dans une méthode thérapeutique chez un sujet, dans laquelle les liposomes ciblant les lymphocytes individuels comprennent à leur surface (i) un ligand spécifique des lymphocytes, dans laquelle le ligand spécifique des lymphocytes est une cytokine qui stimule les lymphocytes ; et (ii) un anticorps ou un fragment d'anticorps qui se lie à CD137, CTLA-4 ou PD-1 ; et dans laquelle ladite population de liposomes ciblant les lymphocytes est administrée au sujet en ayant besoin par une administration systémique répétée.

3. Population de liposomes ciblant les lymphocytes destinée à être utilisée selon l'une quelconque des revendications 1 à 2, dans laquelle les liposomes ciblant les lymphocytes comprennent en outre un agent actif ; par exemple, dans laquelle l'agent actif est encapsulé dans les liposomes ciblant les lymphocytes ; ou dans laquelle l'agent actif est lié à une surface des liposomes ciblant les lymphocytes.

4. Population de liposomes ciblant les lymphocytes destinée à être utilisée selon l'une quelconque des revendications 1 à 3, dans laquelle les liposomes ciblant les lymphocytes ciblent des cellules T endogènes, par exemple dans laquelle les liposomes ciblant les lymphocytes stimulent l'activité et/ou la prolifération de cellules T endogènes ; et/ou dans laquelle les liposomes ciblant les lymphocytes ciblent des cellules T transférées par adoption ou des cellules T modifiées par génie génétique pour exprimer un récepteur des cellules T.

5. Population de liposomes ciblant les lymphocytes destinée à être utilisée selon l'une quelconque des revendications 1 à 4, dans laquelle la cytokine est une IL-2, une IL-7, une IL-15, ou des formes de superagoniste/mutant de celles-ci, ou une protéine de fusion Fc de l'une quelconque des cytokines susdites.

6. Population de liposomes ciblant les lymphocytes destinée à être utilisée selon l'une quelconque des revendications 3 à 5, dans laquelle l'agent actif est une entité chimique, une protéine, un polypeptide, un peptide, un acide nucléique, une particule semblable à un virus, un stéroïde, un protéoglycane, un lipide ou un hydrate de carbone ; et/ou dans laquelle l'agent actif est un agent thérapeutique ; et/ou dans laquelle l'agent actif est un agent qui inhibe l'immunosuppression, par exemple dans laquelle l'agent actif qui inhibe l'immunosuppression est un inhibiteur de la protéine tyrosine phosphatase (PTPase) Shp1/2.

7. Population de liposomes ciblant les lymphocytes destinée à être utilisée selon l'une quelconque des revendications 1 à 6, dans laquelle les liposomes ciblant les lymphocytes sont des liposomes PÉGylés ciblant les lymphocytes ; et/ou dans laquelle les particules ciblant les lymphocytes sont des particules ciblant des lymphocytes à base de polymère.

8. Population de liposomes ciblant les lymphocytes destinée à être utilisée selon l'une quelconque des revendications 1 à 7, dans laquelle l'administration répétée comprend une administration quotidienne, hebdomadaire ou toutes les deux semaines.

9. Population de liposomes ciblant les lymphocytes destinée à être utilisée selon l'une quelconque des revendications 1 à 8, dans laquelle le sujet a un cancer ; et/ou dans laquelle le sujet a une infection.

10. Population de liposomes ciblant les lymphocytes destinée à être utilisée selon l'une quelconque des revendications 1 à 9, dans laquelle le sujet est soumis ou a été soumis à une thérapie cellulaire adoptive.

11. Population de liposomes ciblant les lymphocytes destinée à être utilisée selon l'une quelconque des revendications 1 à 10, dans laquelle l'administration répétée comprend l'administration substantiellement simultanément avec l'administration de cellules T réactives aux tumeurs, et au moins une administration après l'administration de cellules T réactives aux tumeurs.

12. Population de liposomes ciblant les lymphocytes destinée à être utilisée selon l'une quelconque des revendications 1 à 11, dans laquelle le sujet est soumis ou a été soumis à une thérapie cellulaire adoptive et dans laquelle la thérapie cellulaire adoptive comprend l'administration de cellules T CD8+.

13. Population de liposomes ciblant les lymphocytes destinée à être utilisée selon l'une quelconque des revendications 1 à 12, dans laquelle les liposomes sont administrés par voie parentérale ; par exemple dans laquelle les liposomes sont administrés par voie intraveineuse ou sous-cutanée ; telle que dans laquelle les liposomes sont mis en forme pour l'injection sous la forme de doses unitaires ou dans des récipients multidoses.

14. Population de liposomes ciblant les lymphocytes destinée à être utilisée selon l'une quelconque des revendications 1 à 13, dans laquelle les liposomes sont administrés au moins deux fois, au moins 3 fois, au moins 4 fois, au moins 5 fois, au moins 6 fois, au moins 7 fois, au moins 8 fois, ou plus ; et/ou dans laquelle les liposomes sont administrés de manière régulière ou aléatoire espacée dans le temps ; et/ou dans laquelle les liposomes sont administrés à plusieurs jours d'intervalle, ou à plusieurs semaines d'intervalle ou à plusieurs mois d'intervalle ; et/ou dans laquelle les liposomes sont administrés tous les jours, tous les 2 jours, tous les 3 jours, tous les 4 jours, tous les 5 jours, tous les 6 jours, toutes les semaines, toutes les 2 semaines, toutes les 3 semaines, ou toutes les 4 semaines.

15. Population de liposomes ciblant les lymphocytes destinée à être utilisée selon l'une quelconque des revendications 1 à 14, dans laquelle les liposomes sont mis en forme en une composition pharmaceutique sous la forme d'une pluralité de doses.
